# EUROPEAN PATENT SPECIFICATION

(11) **EP 1 691 854 B1**
(45) Date of publication and mention of the grant of the patent: **14.09.2011**
(21) Application number: 04800661.3
(22) Date of filing: 03.11.2004
(51) Int. Cl.: A61L 27/34, A61L 27/44, A61L 27/52, A61L 27/54, A61L 27/58, A61L 29/08, A61L 31/10

(54) **HYDROGEL PROVIDING CELL-SPECIFIC INGROWTH**
HYDROGEL FÜR ZELLSPEZIFISCHES EINWACHSEN
HYDROGEL INDUISANT UNE CROISSANCE INTERNE SPECIFIQUE D'UNE CELLULE

(30) Priority: 03.11.2003 US 517113 P
(43) Date of publication of application: 23.08.2006
(73) Proprietor: Medtronic, Inc., Minneapolis, MN 55432-5604 (US)
(72) Inventor: ZILLA, Peter, Cape Town (ZA); DAVIES, Neil, Vredehoek (ZA); DOWER, Terri, Hout Bay (ZA); BRACHER, Mona, Greenpoint (ZA)
(74) Representative: Hughes, Andrea Michelle
(86) International application number: PCT/US2004/036591
(87) International publication number: WO 2005/042046

(56) References cited:
- WO-A-00/44808
- US-A1- 2003 045 927
- LUTOLF M P ET AL: "Synthetic matrix metalloproteinase-sensitive hydrogels for the conduction of tissue regeneration: Engineering cell-invasion characteristics." PROCEEDINGS OF THE NATIONAL ACADEMY OF SCIENCES OF THE UNITED STATES OF AMERICA, vol. 100, no. 9, 29 April 2003 (2003-04-29), pages 5413-5418, XP002321593 ISSN: 0027-8424 cited in the application & LUTOLF ET AL.: "Supporting Materials and Methods" PNAS, vol. 100, no. 9, 29 April 2003 (2003-04-29), pages 1-5, USA
- CHEN E I ET AL: "Unique substrate recognition profile for matrix metalloproteinase-2" JOURNAL OF BIOLOGICAL CHEMISTRY, AMERICAN SOCIETY OF BIOLOGICAL CHEMISTS, BALTIMORE, MD, US, vol. 277, no. 6, 8 February 2002 (2002-02-08), pages 4485-4491, XP002963031 ISSN: 0021-9258 cited in the application
- KRIDEL S J ET AL: "Substrate hydrolysis by matrix metalloproteinase-9" JOURNAL OF BIOLOGICAL CHEMISTRY, AMERICAN SOCIETY OF BIOLOGICAL CHEMISTS, BALTIMORE, MD, US, vol. 276, no. 23, June 2001 (2001-06), pages 20572-20578, XP002963030 ISSN: 0021-9258 cited in the application
- WONG J Y ET AL: "Identification and validation of a novel cell-recognition site (KNEED) on the 8th type III domain of fibronectin" BIOMATERIALS, ELSEVIER SCIENCE PUBLISHERS BV., BARKING, GB, vol. 23, no. 18, September 2002 (2002-09), pages 3865-3870, XP004372498 ISSN: 0142-9612
- YAMADA YOSHIHIKO ET AL: "Functional domains of cell adhesion molecules" CURRENT OPINION IN CELL BIOLOGY, vol. 4, no. 5, 1992, pages 819-823, XP002321594 ISSN: 0955-0674 cited in the application

### SEQUENCE LISTING

<110> Davies, Neil
<120> HYDROGEL PROVIDING CELL-SPECIFIC INGROWTH
<130> 539.5008
<160> NUMBER OF SEQ ID NOS: 114
<170> PatentIn version 3.2
<210> SEQ ID NO: 1
   <211> 8
   <212> PRT .
   <213> Artificial
<220>
   <223> peptide substrates based on phage clones
<400> 1
   Ser Gly Pro Tyr Val Ile Trp Leu
   1 5
<210> SEQ ID NO: 2
   <211> 8
   <212> PRT
   <213> Artificial
<220>
   <223> peptide substrates based on phage clones
<400> 2
   Ser Gly Pro Val Trp Tyr Met Ser
   1 5
<210> SEQ ID NO: 3
   <211> 8
   <212> PRT
   <213> Artificial
<220>
   <223> peptide substrates based on phage clones
<400> 3
   Ser Gly Pro Val Trp Tyr Met Ser
   1 5
<210> SEQ ID NO: 4
   <211> 8
   <212> PRT
   <213> Artificial
<220>
   <223> peptide substrate based on phage clones
<400> 4
   Ser Gly Pro Val Arg Tyr Met Val
   1 5
<210> SEQ ID NO: 5
   <211> 8
   <212> PRT
   <213> Artificial
<220>
   <223> peptide substrate based on phage clones
<400> 5
   Gly Leu Pro Arg Trp Leu Leu Thr
   1 5
<210> SEQ ID NO: 6
   <211> 8
   <212> PRT
   <213> Artificial
<220>
   <223> peptide substraes based on phage clones
<400> 6
   Gly Arg Pro Ile Arg Met Lys Thr
   1 5
<210> SEQ ID NO: 7
   <211> 8
   <212> PRT
   <213> Artificial
<220>
   <223> peptide substrates based on phage clones
<400> 7
   Leu Arg Leu Ala Ala Ile Thr Ala
   1 5
<210> SEQ ID NO: 8
   <211> 8
   <212> PRT
   <213> Artificial
<220>
   <223> peptide substrates based on phage clones
<400> 8
   Ile Tyr Leu Gly Trp Ala Thr Ala
   1 5
<210> SEQ ID NO: 9
   <211> 8
   <212> PRT
   <213> Artificial
<220>
   <223> peptide substrate based on phage clones
<400> 9
   Pro Met Ile Ser Val Leu Thr Ala
   1 5
<210> SEQ ID NO: 10
   <211> 8
   <212> PRT
   <213> Artificial
<220>
   <223> peptide substrate based on phage clones
<400> 10
   Glu Ser Leu Ala Tyr Tyr Thr Ala
   1 5
<210> SEQ ID NO: 11
   <211> 8
   <212> PRT
   <213> Artificial
<220>
   <223> peptide substrate based on phage clones
<400> 11
   Gly Ser Leu His Ser Ile Ile Thr
   1 5
<210> SEQ ID NO: 12
   <211> 8
   <212> PRT
   <213> Artificial
<220>
   <223> peptide substrate based on phage clones
<400> 12
   Ser Asp Ile Arg Met Leu Thr Ala
   1 5
<210> SEQ ID NO: 13
   <211> 8
   <212> PRT
   <213> Artificial
<220>
   <223> peptide substrate based on phage clones
<400> 13
   Phe Asn Leu Tyr Asn Leu Thr Ala
   1 5
<210> SEQ ID NO: 14
   <211> 8
   <212> PRT
   <213> Artificial
<220>
   <223> peptide substrate based on phage clones
<400> 14
   Gly Tyr Leu Gln Val Leu Leu Thr
   1 5
<210> SEQ ID NO: 15
   <211> 8
   <212> PRT
   <213> Artificial
<220>
   <223> peptide substrate based on phage clones
<400> 15
   Ser Gly Ile Val Asn Leu Tyr Pro
   1 5
<210> SEQ ID NO: 16
   <211> 8
   <212> PRT
   <213> Artificial
<220>
   <223> peptide sequence based on phage clones
<400> 16
   Glu Tyr Leu His Met Arg Thr Ala
   1 5
<210> SEQ ID NO: 17
   <211> 8
   <212> PRT
   <213> Artificial
<220>
   <223> peptide substrates based on phage clones
<400> 17
   Val Gly Leu Ile Ala Ile Thr Ala
   1 5
<210> SEQ ID NO: 18
   <211> 8
   <212> PRT
   <213> Artificial
<220>
   <223> peptide sequence based on phage clones
<400> 18
   Arg Ser Leu Ser Arg Leu Thr Ala
   1 5
<210> SEQ ID NO: 19
   <211> 8
   <212> PRT
   <213> Artificial
<220>
   <223> peptide substrate based on phage clones
<400> 19
   Asn Arg Tyr Ser Ser Leu Thr Ala
   1 5
<210> SEQ ID NO: 20
   <211> 8
   <212> PRT
   <213> Artificial
<220>
   <223> peptide sequence based on phage clones
<400> 20
   Trp Thr Ser Ser Trp Leu Thr Ala
   1 5
<210> SEQ ID NO: 21
   <211> 8
   <212> PRT
   <213> Artificial
<220>
   <223> peptide substrate based on phage clones
<400> 21
   Gly Ala Val Ser Trp Leu Leu Thr
   1 5
<210> SEQ ID NO: 22
   <211> 8
   <212> PRT
   <213> Artificial
<220>
   <223> peptide sequence based on phage clones
<400> 22
   Ala Asn Ile Ser Asp Leu Thr Ala
   1 5
<210> SEQ ID NO: 23
   <211> 8
   <212> PRT
   <213> Artificial
<220>
   <223> peptide sequence based on phage clones
<400> 23
   Thr Ile Leu Ser Leu Leu Thr Ala
   1 5
<210> SEQ ID NO: 24
   <211> 8
   <212> PRT
   <213> Artificial
<220>
   <223> peptide sequence based on phage clones
<400> 24
   Gly Phe Asn Ser Met Leu Lys Thr
   1 5
<210> SEQ ID NO: 25
   <211> 8 .
   <212> PRT
   <213> Artificial
<220>
   <223> peptide sequence based on phage clones
<400> 25
   Gly Leu His Arg Arg Ile Asp Thr
   1 5
<210> SEQ ID NO: 26
   <211> 8
   <212> PRT
   <213> Artificial
<220>
   <223> peptide sequence based on phage clones
<400> 26
   Gly Met His Ser Arg Pro Pro Thr
   1 5
<210> SEQ ID NO: 27
   <211> 8
   <212> PRT
   <213> Artificial
<220>
   <223> peptide sequence based on phage clones
<400> 27
   His Met His Lys Ala Leu Thr Ala
   1 5
<210> SEQ ID NO: 28
   <211> 8
   <212> PRT
   <213> Artificial
<220>
   <223> peptide sequence based on phage clones
<400> 28
   Gly Arg His Leu Gly Leu Gln Thr
   1 5
<210> SEQ ID NO: 29
   <211> 8
   <212> PRT
   <213> Artificial
<220>
   <223> peptide sequence based on phage clones
<400> 29
   Gly Leu His Lys Lys Val His Thr
   1 5
<210> SEQ ID NO: 30
   <211> 8
   <212> PRT
   <213> Artificial
<220>
   <223> peptide sequence based on phage clones
<400> 30
   Gly Ala His Ala Lys His Trp Thr
   1 5
<210> SEQ ID NO: 31
   <211> 10
   <212> PRT
   <213> Artificial
<220>
   <223> peptide substrate based on phage clones
<400> 31
   Ser Gly Ala Lys Pro Arg Ala Leu Thr Ala
   1 5 10
<210> SEQ ID NO: 32
   <211> 10
   <212> PRT
   <213> Artificial
<220>
   <223> peptide substrates based on phage clones
<400> 32
   Ser Gly Leu Arg Leu Ala Ala Ile Thr Ala
   1 5 10
<210> SEQ ID NO: 33
   <211> 10
   <212> PRT
   <213> Artificial
<220>
   <223> peptide substrate based on phage clones
<400> 33
   Ser Gly Glu Ser Leu Ala Tyr Tyr Thr Ala
   1 5 10
<210> SEQ ID NO: 34
   <211> 10
   <212> PRT
   <213> Artificial
<220>
   <223> peptide substrate based on phage clones
<400> 34
   Ser Gly Glu Ser Pro Ala Tyr Tyr Thr Ala
   1 5 10
<210> SEQ ID NO: 35
   <211> 10
   <212> PRT
   <213> Artificial
<220>
   <223> peptide substrate based on phage clones
<400> 35
   Ser Gly Glu Ser Leu Arg Tyr Tyr Thr Ala
   1 5 10
<210> SEQ ID NO: 36
   <211> 10
   <212> PRT
   <213> Artificial
<220>
   <223> peptide substrate based on phage clones
<400> 36
   Ser Gly Arg Ser Leu Ser Arg Leu Thr Ala
   1 5 10
<210> SEQ ID NO: 37
   <211> 10
   <212> PRT
   <213> Artificial
<220>
   <223> peptide substrate based on phage clones
<400> 37
   Ser Gly Arg Ser Leu Arg Arg Leu Thr Ala
   1 5 10
<210> SEQ ID NO: 38
   <211> 10
   <212> PRT
   <213> Artificial
<220>
   <223> peptide sequence based on phage clones
<400> 38
   Ser Gly Ala Val Ser Trp Leu Leu Thr Ala
   1 5 10
<210> SEQ ID NO: 39
   <211> 10
   <212> PRT
   <213> Artificial
<220>
   <223> peptide substrate based on phage clones
<400> 39
   Ser Gly Ala Pro Ser Trp Leu Leu Thr Ala
   1 5 10
<210> SEQ ID NO: 40
   <211> 10
   <212> PRT
   <213> Artificial
<220>
   <223> peptide substrate based on phage clones
<400> 40
   Ser Gly Ala Val Arg Trp Leu Leu Thr Ala
   1 5 10
<210> SEQ ID NO: 41
   <211> 10
   <212> PRT
   <213> Artificial
<220>
   <223> peptide substrate based on phage clones
<400> 41
   Ser Gly Ala Asn Ile Ser Asp Leu Thr Ala
   1 5 10
<210> SEQ ID NO: 42
   <211> 10
   <212> PRT
   <213> Artificial
<220>
   <223> peptide substrate based on phage clones
<400> 42
   Ser Gly Asn Arg Tyr Ser Ser Leu Thr Ala
   1 5 10
<210> SEQ ID NO: 43
   <211> 10
   <212> PRT
   <213> Artificial
<220>
   <223> peptide substrate based on phage clones
<400> 43
   Ser Gly His Met His Lys Ala Leu Thr Ala
   1 5 10
<210> SEQ ID NO: 44
   <211> 10
   <212> PRT
   <213> Artificial
<220>
   <223> peptide substrate based on phage clones
<400> 44
   Ser Gly His Met His Ala Ala Leu Thr Ala
   1 5 10
<210> SEQ ID NO: 45
   <211> 6
   <212> PRT
   <213> Artificial
<220>
   <223> peptide substrate based on phage clones
<400> 45
   Pro Leu Phe Tyr Ser Val
   1 5
<210> SEQ ID NO: 46
   <211> 7
   <212> PRT
   <213> Artificial
<220>
   <223> peptide substrate based on phage clones
<400> 46
   Lys Ile Pro Arg Thr Leu Thr
   1 5
<210> SEQ ID NO: 47
   <211> 6
   <212> PRT
   <213> Artificial
<220>
   <223> peptide substrate based on phage clones
<400> 47
   Pro Leu Arg Leu Ser Trp
   1 5
<210> SEQ ID NO: 48
   <211> 6
   <212> PRT
   <213> Artificial
<220>
   <223> peptide substrates based on phage clones
<400> 48
   Pro Arg Ala Val Ser Thr
   1 5
<210> SEQ ID NO: 49
   <211> 7
   <212> PRT
   <213> Artificial
<220>
   <223> peptide substrates based on phage clones
<400> 49
   Lys Gly Pro Arg Gln Ile Thr
   1 5
<210> SEQ ID NO: 50
   <211> 6
   <212> PRT
   <213> Artificial
<220>
   <223> peptide substrate based on phage clones
<400> 50
   Pro Arg Pro Leu Ser Gly
   1 5
<210> SEQ ID NO: 51
   <211> 6
   <212> PRT
   <213> Artificial
<220>
   <223> peptide substrate based on phage clones
<400> 51
   Trp Pro Leu Gly Leu Ala
   1 5
<210> SEQ ID NO: 52
   <211> 7
   <212> PRT
   <213> Artificial
<220>
   <223> peptide substrate based on phage clones
<400> 52
   Phe Arg Pro Arg Ser Ile Thr
   1 5
<210> SEQ ID NO: 53
   <211> 7
   <212> PRT
   <213> Artificial
<220>
   <223> peptide substrate based on phage clones
<400> 53
   Arg Leu Pro Val Gly Leu Thr
   1 5
<210> SEQ ID NO: 54
   <211> 7
   <212> PRT
   <213> Artificial
<220>
   <223> peptide substrates based on phage clones
<400> 54
   Arg Ser Pro Lys Ser Leu Thr
   1 5
<210> SEQ ID NO: 55
   <211> 6
   <212> PRT
   <213> Artificial
<220>
   <223> peptide substrates based on phage clones
<400> 55
   Pro Val Trp Leu Ala Ala
   1 5
<210> SEQ ID NO: 56
   <211> 8
   <212> PRT
   <213> Artificial
<220>
   <223> peptide substrates based on phage clones
<400> 56
   Ile His Pro Ser Ser Leu Thr Ala
   1 5
<210> SEQ ID NO: 57
   <211> 6
   <212> PRT
   <213> Artificial
<220>
   <223> peptide substrates based on phage clones
<400> 57
   Pro Ala Ser Phe Thr Ser
   1 5
<210> SEQ ID NO: 58
   <210>
   <211> 7
   <212> PRT
   <213> Artificial
<220>
   <223> peptide substrates based on phage clones
<400> 58
<210> SEQ ID NO: 59
   <211> 7
   <212> PRT
   <213> Artificial
<220>
   <223> peptide substrates based on phage clones
<400> 59
   Met Lys Pro Ala Ser Trp Thr
   1 5
<210> SEQ ID NO: 60
   <211> 7
   <212> PRT
   <213> Artificial
<220>
   <223> peptide substrates based on phage clones
<400> 60
   Thr His Pro Tyr Thr Met Thr
   1 5
<210> SEQ ID NO: 61
   <211> 7
   <212> PRT
   <213> Artificial
<220>
   <223> peptide substrate based on phage clones
<400> 61
   Thr Pro Ala Tyr Met Leu Thr
   1 5
<210> SEQ ID NO: 62
   <211> 5
   <212> PRT
   <213> Artificial
<220>
   <223> peptide substrate based on phage clones
<400> 62
   Pro Leu Tyr Leu Thr
   1 5
<210> SEQ ID NO: 63
   <211> 6
   <212> PRT
   <213> Artificial
<220>
   <223> peptide substrate based on phage clones
<400> 63
   Pro Gly Leu Ile Gly Thr
   1 5
<210> SEQ ID NO: 64
   <211> 6
   <212> PRT
   <213> Artificial
<220>
   <223> peptide substrate based on phage clones
<400> 64
   Pro Arg Ser Ile Ser Asn
   1 5
<210> SEQ ID NO: 65
   <211> 7
   <212> PRT
   <213> Artificial
<220>
   <223> peptide substrate based on phage clones
<400> 65
   Arg Leu Pro Ala Ser Tyr Thr
   1 5
<210> SEQ ID NO: 66
   <211> 7
   <212> PRT
   <213> Artificial
<220>
   <223> peptide substrate based on phage clones
<400> 66
   Asn Pro Pro Arg Tyr Leu Thr
   1 5
<210> SEQ ID NO: 67
   <211> 6
   <212> PRT
   <213> Artificial
<220>
   <223> peptide substrate based on phage clones
<400> 67
   Pro Lys Thr Gln Ile Ser
   1 5
<210> SEQ ID NO: 68
   <211> 7
   <212> PRT
   <213> Artificial
<220>
   <223> peptide substrate based on phage clones
<400> 68
   Ser Val Pro Arg His Phe Thr
   1 5
<210> SEQ ID NO: 69
   <211> 7
   <212> PRT
   <213> Artificial
<220>
   <223> peptide substrate based on phage clones
<400> 69
   Leu Leu Pro Ala Trp Leu Thr
   1 5
<210> SEQ ID NO: 70
   <211> 7
   <212> PRT
   <213> Artificial
<220>
   <223> peptide substrate based on phage clones
<400> 70
   Thr His Pro Tyr Thr Met Thr
   1 5
<210> SEQ ID NO: 71
   <211> 7
   <212> PRT
   <213> Artificial
<220>
   <223> peptide substrate based on phage clones
<400> 71
   Leu Arg Pro Ala Lys Ser Thr
   1 5
<210> SEQ ID NO:72
   <211> 7
   <212> PRT
   <213> Artificial
<220>
   <223> peptide substrate based on phage clones
<400> 72
   Ser Gly Pro Ser Thr Ser Thr
   1 5
<210> SEQ ID NO: 73
   <211> 6
   <212> PRT
   <213> Artificial
<220>
   <223> peptide substrate based on phage clones
<400> 73
   Gly Ser Gly Leu Lys Ala
   1 5
<210> SEQ ID NO: 74
   <211> 6
   <212> PRT
   <213> Artificial
<220>
   <223> peptide substrate based on phage clones
<400> 74
   Ala Met Gly Leu Lys Ser
   1 5
<210> SEQ ID NO: 75
   <211> 6
   <212> PRT
   <213> Artificial
<220>
   <223> peptide substrate based on phage clones
<400> 75
   Lys Val Gly Leu Arg Thr
   1 5
<210> SEQ ID NO: 76
   <211> 7
   <212> PRT
   <213> Artificial
<220>
   <223> peptide substrate based on phage clones
<400> 76
   Gly Arg Arg Leu Ile His His
   1 5
<210> SEQ ID NO:77
   <211> 7
   <212> PRT
   <213> Artificial
<220>
   <223> peptide substrate based on phage clones
<400> 77
   His Pro Arg Arg Ser Ile Thr
   1 5
<210> SEQ ID NO: 78
   <211> 7
   <212> PRT
   <213> Artificial
<220>
   <223> peptide substrate based on phage clones
<400> 78
   Gly Arg Arg Leu Leu Ser Arg
   1 5
<210> SEQ ID NO: 79
   <211> 7
   <212> PRT
   <213> Artificial
<220>
   <223> peptide substrate based on phage clones
<400> 79
   Ala Leu Arg Arg Leu Glu Thr
   1 5
<210> SEQ ID NO: 80
   <211> 7
   <212> PRT
   <213> Artificial
<220>
   <223> peptide substrate based on phage clones
<400> 80
   Phe Tyr Lys Arg Val Leu Thr
   1 5
<210> SEQ ID NO: 81
   <211> 6
   <212> PRT
   <213> Artificial
<220>
   <223> peptide substrate based on phage clones
<400> 81
   Phe Arg Arg Ile Cys Val
   1 5
<210> SEQ ID NO: 82
   <211> 8
   <212> PRT
   <213> Artificial
<220>
   <223> peptide substrates based on phage clones
<400> 82
   Val Phe Phe Arg Arg Gln Thr Ala
   1 5
<210> SEQ ID NO: 83
   <211> 8
   <212> PRT
   <213> Artificial
<220>
   <223> peptide substrate based on phage clones
<400> 83
   Gly Leu Ala Arg Asn Ile Thr Ala
   1 5
<210> SEQ ID NO: 84
   <211> 8
   <212> PRT
   <213> Artificial
<220>
   <223> peptide substrate based on phage clones
<400> 84
   Phe Gly Ser Arg Tyr Leu Thr Ala
   1 5
<210> SEQ ID NO: 85
   <211> 7
   <212> PRT
   <213> Artificial
<220>
   <223> peptide substrate based on phage clones
<400> 85
   Gln Asp Arg Tyr Leu Asn Thr
   1 5
<210> SEQ ID NO: 86
   <211> 10
   <212> PRT
   <213> Artificial
<220>
   <223> peptide substrate based on phage clones
<400> 86
   Ser Gly Lys Gly Pro Arg Gln Ile Thr Ala
   1 5 10
<210> SEQ ID NO: 87
   <211> 10
   <212> PRT
   <213> Artificial
<220>
   <223> peptide substratebased on phage clones
<400> 87
   Ser Gly Lys Ile Pro Arg Thr Leu Thr Ala
   1 5 10
<210> SEQ ID NO: 88
   <211> 10
   <212> PRT
   <213> Artificial
<220>
   <223> peptide substrate based on phage clones
<400> 88
   Ser Gly Pro Arg Ala Val Ser Thr Thr Ala
   1 5 10
<210> SEQ ID NO: 89
   <211> 10
   <212> PRT
   <213> Artificial
<220>
   <223> peptide substrate based on phage clones
<400> 89
   Ser Gly Lys Ile Pro Arg Arg Leu Thr Ala
   1 5 10
<210> SEQ ID NO: 90
   <211> 10
   <212> PRT
   <213> Artificial
<220>
   <223> peptide substrate based on phage clones
<400> 90
   Ser Gly Lys Ile Pro Arg Thr Ala Thr Ala
   1 5 10
<210> SEQ ID NO: 91
   <211> 10
   <212> PRT
   <213> Artificial
<220>
   <223> peptide substrate based on phage clones
<400> 91
   Ser Gly Pro Leu Phe Tyr Ser Val Thr Ala
   1 5 10
<210> SEQ ID NO: 92
   <211> 10
   <212> PRT
   <213> Artificial
<220>
   <223> peptide substrate based on phage clones
<400> 92
   Ser Gly Gln Pro His Tyr Leu Thr Thr Ala
   1 5 10
<210> SEQ ID NO: 93
   <211> 10
   <212> PRT
   <213> Artificial
<220>
   <223> peptide substrate based on phage clones
<400> 93
   Ser Gly Leu Arg Pro Ala Lys Ser Thr Ala
   1 5 10
<210> SEQ ID NO: 94
   <211> 10
   <212> PRT
   <213> Artificial
<220>
   <223> peptide substrate based on phage clones
<400> 94
   Ser Gly Leu Lys Ala Leu Met Ile Thr Ala
   1 5 10
<210> SEQ ID NO: 95
   <211> 10
   <212> PRT
   <213> Artificial
<220>
   <223> peptide substrate based on phage clones
<400> 95
   Ser Gly Phe Gly Ser Arg Tyr Leu Thr Ala
   1 5 10
<210> SEQ ID NO: 96
   <211> 10
   <212> PRT
   <213> Artificial
<220>
   <223> peptide substrates based on phage clones
<400> 96
   Ser Gly Arg Arg Leu Leu Ser Arg Thr Ala
   1 5 10
<210> SEQ ID NO: 97
   <211> 10
   <212> PRT
   <213> Artificial
<220>
   <223> peptide substrates based on phage clones
<400> 97
   Ser Gly Arg Arg Leu Ile His His Thr Ala
   1 5 10
<210> SEQ ID NO: 98
   <211> 4
   <212> PRT
   <213> Unknown
<220>
   <223> extracellular matrix protein cell binding domain
<400> 98
   Arg Gly Asp Ser
   1
<210> SEQ ID NO: 99
   <211> 4
   <212> PRT
   <213> Unknown
<220>
   <223> extracellular matrix binding domain
<400> 99
   Arg Glu Asp Val
   1
<210> SEQ ID NO: 100
   <211> 4
   <212> PRT
   <213> Unknown
<220>
   <223> extracellular matrix protein binding domain
<400> 100
   Arg Gly Asp Val
   1
<210> SEQ ID NO: 101
   <211> 5
   <212> PRT
   <213> Unknown
<220>
   <223> extracellular matrix protein binding domain
<400> 101
   Leu Arg Gly Asp Asn
   1 5
<210> SEQ ID NO: 102
   <211> 5
   <212> PRT
   <213> Unknown
<220>
   <223> extracellular matrix protein binding domain
<400> 102
   Ile Lys Val Ala Val
   1 5
<210> SEQ ID NO: 103
   <211> 5
   <212> PRT
   <213> Unknown
<220>
   <223> extracellular matrix protein binding domain
<400> 103
   Tyr Ile Gly Ser Arg
   1 5
<210> SEQ ID NO: 104
   <211> 5
   <212> PRT
   <213> Unknown
<220>
   <223> extracellular matrix protein binding domain
<400> 104
   Pro Asp Ser Gly Arg
   1 5
<210> SEQ ID NO: 105
   <211> 10
   <212> PRT
   <213> Unknown
<220>
   <223> extracellular matrix protein binding domain
<400> 105
   Arg Asn Ile Ala Glu Ile Ile Lys Asp Ala
   1 5 10
<210> SEQ ID NO: 106
   <211> 4
   <212> PRT
   <213> Unknown
<220>
   <223> extracellular matrix protein binding domain
<400> 106
   Arg Gly Asp Thr
   1
<210> SEQ ID NO: 107
   <211> 4
   <212> PRT
   <213> Unknown
<220>
   <223> extracellular matrix protein binding domain
<400> 107
   Asp Gly Glu Ala
   1
<210> SEQ ID NO: 108
   <211> 4
   <212> PRT
   <213> Unknown
<220>
   <223> extracellular matrix protein binding domain
<220>
   <221> misc_feature
   <222> (3)..(3)
   <223> Xaa can be any naturally occurring amino acid
<400> 108
   Val Thr Xaa Gly
   1
<210> SEQ ID NO: 109
   <211> 5
   <212> PRT
   <213> Unknown
<220>
   <223> extracellular matrix protein binding domain
<400> 109
   Pro His Ser Arg Asn
   1 5
<210> SEQ ID NO: 110
   <211> 11
   <212> PRT
   <213> Artificial
<220>
   <223> synthetic construct
<400> 110
   Gly Cys Gly Gly Gly Arg Gly Asp Ser Pro Gly
   1 5 10
<210> SEQ ID NO: 111
   <211> 13
   <212> PRT
   <213> Artificial
<220>
   <223> synthetic construct
<400> 111
   Gly Cys Gly Gly Gly Val Pro His Ser Arg Asn Ser Gly
   1 5 10
<210> SEQ ID NO: 112
   <211> 11
   <212> PRT
   <213> Artificial
<220>
   <223> synthetic construct
<400> 112
   Gly Cys Gly Gly Gly Tyr Ile Gly Ser Arg Gly
   1 5 10
<210> SEQ ID NO: 113
   <211> 18
   <212> PRT
   <213> Artificial
<220>
   <223> synthetic construct
<400> 113
   Gly Cys Arg Asp Ser Gly Glu Ser Leu Ala Tyr Tyr Thr Ala Asp Arg
   1 5 10 15
   Cys Gly
<210> SEQ ID NO: 114
   <211> 11
   <212> PRT
   <213> Artificial
<220>
   <223> synthetic construct
<400> 114
   Gly Cys Lys Gly Pro Arg Gln Ile Thr Cys Gly
   1 5 10

## Description

### Cross-Reference to Related Applications

This application claims priority to U.S. Provisional Application Serial No. 60/517,113, titled "Hydrogel Providing Cell-Specific Ingrowth", filed on November 3, 2003.

### Field of the Invention

The present invention relates to biocompatible polymers that can be applied to biological and non-biological surfaces to encourage ingrowth of specific cell types. More particularly, the present invention relates to hydrogels derivatized with short peptide sequences that encourage ingrowth of specific cell types and, optionally, encourage adhesion of such cell types.

### Background of the Invention

There is a need for biomaterials that can be used to facilitate the integration of implantable medical devices. Biomaterials are used in medical implants, extracorporeal devices, and disposable items utilized in medicine, surgery, dentistry, and veterinary medicine. The traditional approach for implantable medical devices has been to make the device from, or coat the device with, material that is biocompatible. Generally, the prime requirement for a biocompatible material has been that it remain inert and do no harm when placed within the body. Relatively inert materials used to construct implantable medical devices include certain metals, polymers, ceramics, and composites. For example, metal alloys have been developed that provide improved physical and chemical properties, such as strength, durability and corrosion resistance.

Various polymer coatings have been devised to provide an inert surface for medical devices including silicones, polyurethanes, thermoplastic elastomers, fluoropolymers, and various biodegradable polymers such as polylactides and polyglycolides. Coating a medical device with polymeric material may make the device more biocompatible by providing a surface layer of material that is extremely unreactive. A polymer used as a coating material can be optimized to provide an unreactive surface, as it does not need to have the physical strength or other functional aspects of the material used to construct the device.

Hydrogels are a type of biocompatible polymer that provide several advantages. Hydrogels are colloids in which a polymer is combined with water to produce a viscous, jellylike product Polyvinyl alcohol and polyethylene glycol are examples of two polymers that readily form hydrogels. Hydrogels are generally inexpensive, and are biodegradable while not releasing potentially toxic degradation products. They are also notably inert and lack adhesiveness for cells. U.S. Patents Nos. 5,573,934 and 5,626,863 to Hubbell et al. disclose the use of hydrogel materials including biodegradable polymers such as polylactide and polyglycolide, terminated with photopolymerizable groups such as acrylates. See also Lutolf et al., "Synthetic Matrix Metalloproteinase-Sensitive Hydrogels for the Conduction of Tissue Regeneration: Engineering Cell-Invasion Characteristics" PNAS 100, 9/ 5413-5418 (2003). These materials can be applied to a tissue surface and polymerized to form tissue coatings, and are useful for inhibiting adhesion and immune recognition. Hydrogels also exhibit high volume, making them useful as reservoirs for drugs or other materials. When used as a drug reservoir, the hydrogel essentially functions as a drug delivery system. An example of drug delivery by a hydrogel is found in U.S. Patent 6,228,393 to DiCosmo et al., in which hydrogels used to coat medical devices are loaded with therapeutic agents such as antibiotics.

Recent studies have shown, however, that inert biomaterials may not provide the desired response when used in implantable medical devices. Advances in molecular biology and biological surface science indicate that even inert material will eventually provoke a response, such as inflammation or fibrous encapsulation, as the inert material is not properly integrated with the tissues within the body. A variety of processes occur at the molecular level at the surface of an implant, depending on the nature of the surface. For a traditional metal implant, these processes include metal/metal oxide ion diffusion, biological ion incorporation, adsorption /desorption of biological molecules, and conformational changes of adhered biological molecules. All of these processes can eventually lead to an inflammation, coagulation, or other undesirable responses to the implanted material. Similar processes occur even when sophisticated materials such as the hydrogel polymers are used.

More recently, investigators have been striving to do more than simply make biomaterials that are inert. The new approach to biomaterial design aims to provide material that is incorporated into the living system in which it is placed. Rather than striving to be ignored by the body, these materials attempt to integrate themselves into the surrounding tissue.

The extracellular matrix is a network of proteins and polysaccharides that forms a dynamic layer of material between cells. It plays an important role in regulating tumor growth by inhibiting undesirable cell proliferation, while encouraging appropriate cell proliferation during wound healing. As such, it is a locus of growth factor production and accumulation. It also provides the substrate to which anchorage-dependent cells need to firmly adhere; an event that is a prerequisite for many important cell functions such as migration and cellular protein synthesis. Cell interactions with the extracellular matrix may regulate cell functions via receptor-mediated signaling mechanisms and/or by modulating the cell response to growth factors.

Two phenomena occurring in the formation and maintenance of tissue are cell migration and cell adhesion., Both processes are controlled in large part by the extracellular matrix. Essentially, cell migration is required to place desirable cells at a specific location, while cell adhesion maintains them there. Cell migration may be directed by secretion of a soluble chemical that attracts migrating cells, or by the laying down of adhesive molecules in the extracellular matrix to guide migrating cells to the desired location. Once adjacent to the appropriate cells, proteins such as intercellular adhesion molecules (ICAMs) and cadherins that are present on the surfaces of cells mediate cell-to-cell adhesion. These processes are particularly important in embryonic development and wound healing.

The extracellular matrix contains a number of noncollagen adhesive proteins that typically have multiple domains, each with specific binding sites for other matrix macromolecules and for receptors on the surface of cells. Fibronectin is an important adhesive protein, consisting of two very large subunits joined by a pair of disulfide bonds. Each subunit is folded into a series of distinct rod-like domains, that consist of smaller modules, each of which is serially repeated. These serially repeated modules are known to be involved in binding to cells. Fibronectin also plays an important role in regulating cell migration. Another protein involved in cell adhesion is laminin, which together with several other proteins makes up the basal laminae; a mat of specialized extracellular matrix that underlies epithelial cells. The basal laminae is involved in cell differentiation, cell migration, and wound repair.

The proteins that bind to extracellular matrix adhesive proteins such as fibronectin and laminin are integrins, which are specialized cell-surface receptors. While some integrins bind only one adhesive protein, for example fibronectin or laminin, others bind more than one. The same integrin molecule, located in different cell types, can display different ligand-binding activity, indicating that additional factors can interact with integrins to modulate their binding activity. Integrins function as transmembrane linkers mediating the interactions between the cytoskeleton and the extracellular matrix that are required for cells to grip the matrix. Most integrins connect to bundles of actin filaments. Following the binding of a typical integrin to its ligand in the matrix, integrin initiates the assembly of a complex of intracellular attachment proteins that link the integrin to actin filaments in the cell vortex. This is thought to be how focal adhesion sites form between cells and the extracellular matrix.

One means to promote a positive interaction between an implanted device and the *in vivo* environment is to derivatize the surface of the device with peptides in order to promote cell adhesion. Such an approach is described in U.S. Patent 5,278,063 to Hubbell et al.*,* in which polymeric, ceramic, and glass surfaces were derivatized with short peptides in order to promote cell adhesion and the formation of focal contacts.

Another means of promoting a positive interaction between an implanted surface and the *in vivo* environment is to encourage beneficial ingrowth, which is the migration of desirable cells into the biomaterial. A multi-zone, multifunctional graft having a non-thrombogenic and antiproliferative inner zone and an outer zone that encourages beneficial ingrowth and sealing of the graft is described in U.S. Patent 6,440,166 to Kolluri et al. The outer layer described in Kolluri *et al.,* includes growth agents that encourage the infiltration of cells from the exterior environment into the wall of the graft. Examples of growth agents used by Kolluri *et al*. include fibronectin, laminin, and bovine collagen. These proteins, while encouraging ingrowth, do not have a significant selective effect on particular cell types.

Examples of biomaterials, e.g. polymeric hydrogels, formed by nucleophilic addition reaction to conjugated unsaturated groups are disclosed in WO 00/44808 (Hubbell JA). The biomaterials of WO 00/44808 may include cell adhesion sites and protease substrate sites, may provide for the delivery of protein drugs and can be used as medical implants. However, WO 00/44808 does riot disclose a polymer matrix which comprises protease-selective cross-links and thus the biomaterials of WO 00/44808 are not capable of being selectively degraded by desired cell types.

The art would benefit from a biomaterial that can be tailored to encourage the ingrowth and optionally the adhesion of specific cell types in order to encourage the integration of implanted medical devices and scaffolds constructed from or coated with this biomaterial.

### Summary of the Invention

The present invention provides a new biomaterial that can be used to encourage the integration of implantable medical devices by facilitating the ingrowth and optionally the adhesion of specific cells into the biomaterial. This novel biomaterial consists of a hydrophilic polymer, such as a hydrogel, to which small peptides are attached that contain amino acid sequences that encourage the ingrowth of desired cells into the biomaterial The techniques disclosed herein provide for the construction of a degradable polymer including short peptide sequence that crosslink the hydrophilic polymer. Also disclosed are methods for selecting peptide sequences that encourage the selective ingrowth of desired cells. Desired cell types may be, for example, endothelial and smooth muscle cells whose ingrowth integrates the biomaterial into the surrounding tissue and provides proper healing and a positive long-term outcome.

In accordance with one aspect of the invention, a polymer matrix capable of being degraded by a desired predetermined cell type is provided. The matrix comprises a hydrophilic polymer crosslinked by a peptide sequence including a protease cleavage site, which cleavage site is a substrate for a matrix metalloproteinase present in the predetermined cell type but absent or present in much lower levels in non-desired cells. The protease cleavage site is one which is selectively cleaved by the matrix metalloproteinase present in the predetermined cell type. The polymer matrix may also be provided with an appended peptide sequence containing an adhesion site that increases the affinity of the polymer matrix for the predetermined cell type.

In accordance with another aspect of the invention, a biocompatible medical device is provided that includes an implantable medical device having a surface at least partially coated with a polymer matrix of the invention.

In particular embodiments of the invention, the implantable medical device may be a vascular graft, a heart valve, or a sewing ring. In further embodiments, the polymer matrix may include an appended peptide sequence including an adhesion site that increase the affinity of the polymer matrix for the cell type and/or the protease cleavage site is a substrate for a matrix metalloproteinase present in a cell type that promotes tissue healing by ingrowth into the polymer matrix. The tissue healing enabled by the polymer matrix may include transmural vascularization of the implanted medical device. In additional embodiments, the polymer matrix may include a biologically active agent.

In accordance with another aspect of the invention, an implantable film comprising a polymer matrix of the invention, especially a sheet of polymer matrix material capable of being degraded by a predetermined cell type, is provided. Optionally, the polymer matrix further includes an appended peptide sequence containing an adhesion site that increases the affinity of the polymer matrix, for the predetermined cell type. In particular embodiments, the film is capable of preventing the formation of surgical adhesions and may be a tissue scaffold. The protease cleavage site in embodiments of the implantable film may be a substrate for matrix metalloproteinase present in a cell type that promotes tissue healing by ingrowth into the polymer matrix. In some embodiments of the implantable film, the polymer matrix may include a biologically active agent.

In accordance with yet another aspect of the invention, a method is provided for preparing an implantable medical device capable of integrating into a mammalian body by encouraging ingrowth by a predetermined cell type. This method includes the steps of preparing a polymer matrix of the invention by crosslinking a hydrophilic polymer by a peptide sequence including a protease cleavage site, which cleavage site is chosen because it is a substrate for a matrix metalloproteinase present in said cell type, and then coating the polymer matrix on at least a portion of the surface of a medical device. Optionally, the method further includes the step of providing the polymer matrix with appended peptide sequences containing an adhesion site that increases the affinity of the polymer matrix for the predetermined cell type.

In a further embodiment, the invention provides, in combination, a polymer matrix of the invention and cells of said predetermined cell type in contact with said polymer matrix.

In yet a further embodiment, the invention provides a method of providing cell-specific ingrowth of cells into a polymer matrix in which cells of a predetermined cell type are contacted with a polymer matrix of the invention, with the proviso that the method is not a method for the treatment of the human or animal body by surgery or therapy or a diagnostic method carried out on the human or animal body.

The combined use of adherence-inducing and protease-specific peptides in the present invention provides unique advantages. Attraction of cell types by extracellular matrix protein peptide sequences acts synergistically with the use of cross-linking protease-specific proteins to facilitate cell-specific ingrowth. Attraction and adherence of cell types increases the likelihood that those cells will be present to utilize their proteases to degrade and enter into the polymer matrix. As the adhesive peptides are also found within the polymer, they encourage migration and adherence by the desired cells subsequent to ingrowth. Once within the polymer matrix, preferred cells are also exposed to mechanical stresses such as pulsatile stretching of a graft that simulate cell growth, further increasing the number of desired cells within the biomaterial.

### Brief Description of the Drawing

The above and other objects and aspects of the invention are further revealed by the embodiments illustrated by the following accompanying drawings:
FIG. 1 is a diagram representing a polymer matrix derivatized with protease substrates and adhesion sites;
FIG. 2 is a zymogram developed to determine the levels of proteases secreted by specific cell types cultured on various protein coatings

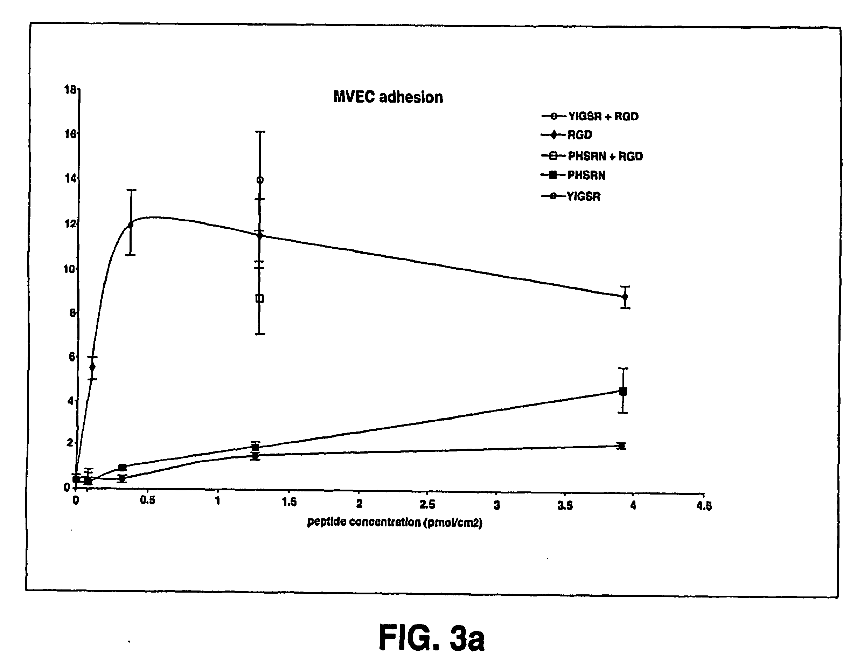

FIG. 3a is a graph showing the adhesion of endothelial cells to peptide-derivatized hydrogel over varying concentrations of peptides.

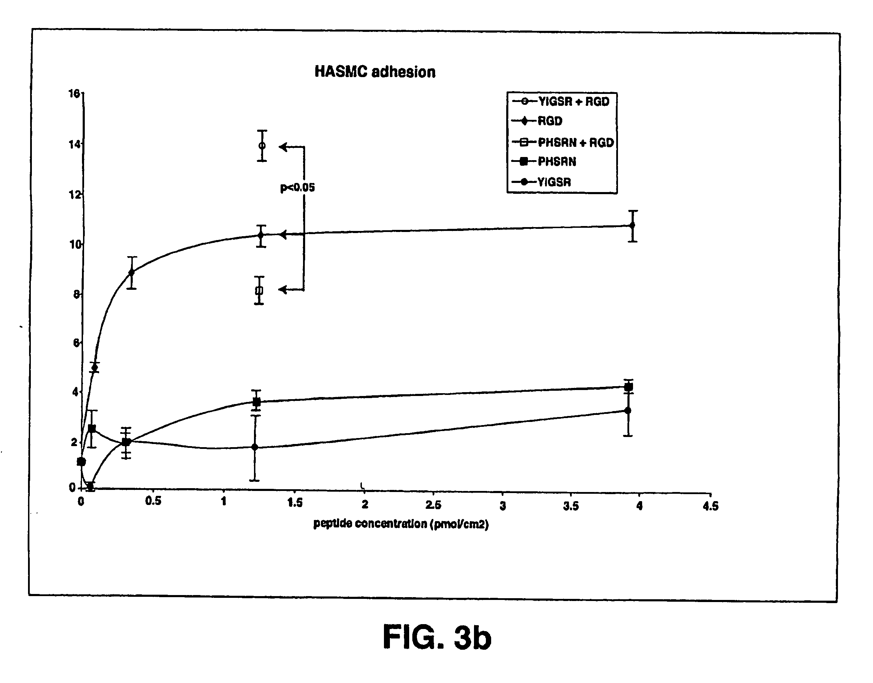

FIG. 3b is a graph showing the adhesion of smooth muscle cells to peptide-derivatized hydrogel over varying concentrations of peptides.
FIG. 4 is a bar graph illustrating the migration speeds of endothelial cells and smooth muscle cells on peptide-derivatized hydrogels.

### Detailed Description of the Preferred Embodiments

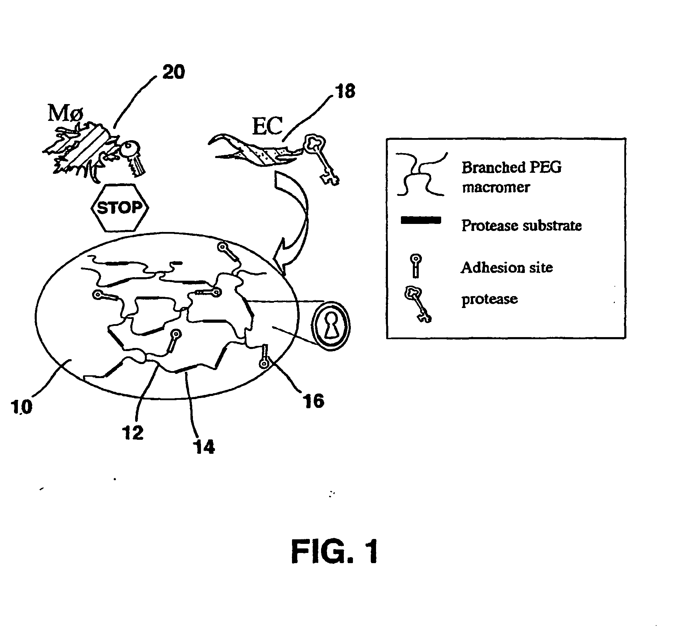

The general nature and function of the present invention are illustrated in FIG. 1 which provides a diagram representing the polymer matrix derivatized with protease substrates and adhesion sites. The polymeric matrix 10 contains a polymer and peptide that form a biomaterial of the present invention. Making up the polymeric matrix 10 are a hydrophilic polymer 12 and peptide sequences derivatized to the polymer. The peptide sequences can be either protease substrates 14 or adhesion sites 16 or both. Protease substrates 14 are short peptide sequences that are used to crosslink the hydrophilic polymer 12 and are selected to be degraded by specific proteases present in the cell type 18. The cell type 18 is predetermined prior to preparation of the polymeric matrix, and is thus sometimes referred to herein as the predetermined cell type. Endothelial cells (EC), shown at 18 in FIG. 1, are one preferred cell type 18.

Adhesion sites 16 are short peptide sequences that are derivatized to the polymer and serve to encourage the migration and adhesion of a cell type 18. These adhesion sites 16 are present on the surface of the polymeric matrix 10 and are also present in the interior, where they serve to secure and encourage the ingrowth of the cell type 18 subsequent to degradation of the protease substrates 14. Ingrowth of a cell type 18, indicated by an arrow in this figure, is encouraged by the nature of the polymer matrix 10, whereas non-preferred cell types 20 such as macrophages are barred from penetrating.

As can be seen from FIG. 1, the polymeric matrix 10 includes a hydrophilic polymer 12 and protease substrates 14 positioned to crosslink the polymer. The polymer matrix 10 may also include adhesion sites 16. These aspects of the invention are discussed in further detail below.

### Biomaterial crosslinked with Protease-susceptible peptides

The biomaterial of the present invention utilizes peptide strands to crosslink the hydrophilic polymer 12. These crosslinking strands provide an important structural component of the polymer matrix 10, and their cleavage weakens the polymer matrix 10 and allows invasion by cells capable of causing further cleavage. The peptide strands used to crosslink the polymer have the amino acid sequence of substrates for specific proteases, which is why they are referred to herein as protease substrates 14. By choosing protease substrates 14 with particular amino acid sequences and incorporating them into the matrix, a polymer matrix is created that is selectively cleaved by specific proteases. Thus, instead of merely being degradable, the biomaterial of the present invention may serve as a selective barrier, allowing penetration only by cells carrying the appropriate protease. The polymer matrix of the present invention comprises peptide strands which are substrates for a matrix metalloproteinase.

A protease, or proteinase, as used herein, is an enzyme that catalytically cleaves peptide bonds that link adjacent amino acids. Proteases are classified into a variety of groups, including serine, cysteine, aspartic and metalloproteinases, based on their catalytic mechanism and substrate specificity. Matrix metalloproteinases, or MMPs, are one class of protease, named for their dependence on metal ions for catalytic activity and their ability to degrade structural proteins of the extracellular matrix. MMPs influence diverse physiological processes, including embryonic development, tissue morphogenesis, wound repair, inflammatory disease, and cancer. See M. D. Sternlicht and Z. Werb, "How Matrix Metalloproteinases regulate cell behavior" Ann. Rev. Cell. Dev. Biol. 17,463 (2001).

It is understood that 25 vertebrate MMPs have been identified, each of which has a distinct substrate specificity.

The inventors have observed that grafts implanted in baboons show extensive ingrowth by inflammatory cells such as macrophages and neutrophils. The early arrival of inflammatory cells is part of the normal physiological response to a wound. Once these cells arrive, inflammation and the formation of foreign body giant cells occurs, both of which are generally undesirable effects after implantation of a medical device or scaffold. Cell types desired to be present in this situation include endothelial cells and smooth muscle cells, cells involved in the integration of healthy tissue. Different cells within the body contain different types and levels of protease, including MMPs. Thus, the polymer matrix utilizes a peptide sequence or sequences cleaved by protease present in desired cells such as endothelial cells and smooth muscle cells, but absent or present in much lower levels in non-desired cells such as macrophages and neutrophils. While the present embodiment focuses on the use of peptide-containing polymer matrices that encourage ingrowth by endothelial and smooth muscle cells, the present invention includes any polymer matrix biomaterial that provides preferential ingrowth by preferred cells, and thus may be utilized to encourage the ingrowth of a variety of different cell types depending on the particular placement and use of the biomaterial within the body.

In choosing a protease substrate 14 that results in preferential ingrowth, it is first determined what proteases are present in the desired cell types. Several molecular techniques have been used to identify and characterize proteases in cells and tissues. For example, Northern blot analysis and reverse trameription-polymerase chain reaction (RT-PCR) have been used to quantitate protease mRNA in cell and tissue extracts, and *in situ* hybridization has been used to localize mRNA expression at the cellular level in tissue sections. One limitation of these techniques is that transcriptional activity does not necessarily reflect the amount and activity of the protein product of a particular gene due to regulation of translational activity and various other protein regulation mechanisms. Western blot and immunohistochemistry analysis may be used to evaluate expression of protease protein levels rather than mRNA. Western blot analysis was conducted to determine the levels of MMP-3 secreted by human Endothelial cells (EC), smooth muscle cells (SMC), fibroblasts (Fb), and macrophages (MΦ), as these are the principle cell types involved in the host response to synthetic implanted materials such as vascular grafts. Western blot analysis indicates that endothelial cells and macrophages do not secrete MMP-3, while fibroblasts, and to a lesser extent, smooth muscle cells, do. Western blot analysis may be used to evaluate the levels of any protease so long as the corresponding antibody is available.

A disadvantage of Western blot analysis to determine protease levels is that many proteases are synthesized in an inactive or pro-enzyme form called a zymogen that requires proteolytic processing to become active, and therefore, such proteases may not be accurately evaluated using the Western blot analysis. Most MMPs are, in fact, produced and secreted as zymogens that require proteolytic cleavage of a pro-peptide domain to be activated. Most antibodies available for immunohistochemical analysis do not discriminate between the active and inactive forms of these enzymes. For this reason, gel enzymography, more commonly referred to as zymography, has been developed to detect and quantify various protease activities and is particularly useful in identifying proteases present in particular cell types for use with this invention. A review of zymography describes the technique in more detail; see Lantz, M., Ciborowski P., "Zymographic techniques for detection and characterization of microbial proteases" Methods Enzymol. 1, 235, 563-94 (1994).
Zymography is a versatile, two-stage technique involving protein separation by electrophoresis followed by detection of proteolytic activity through creation of zones of lysis. It utilizes an SDS polyacrylamide gel where protein degradation is detected on the basis of protein binding dye exclusion. Since activity is detected as part of the assay, inactive zymogen forms are automatically screened out.

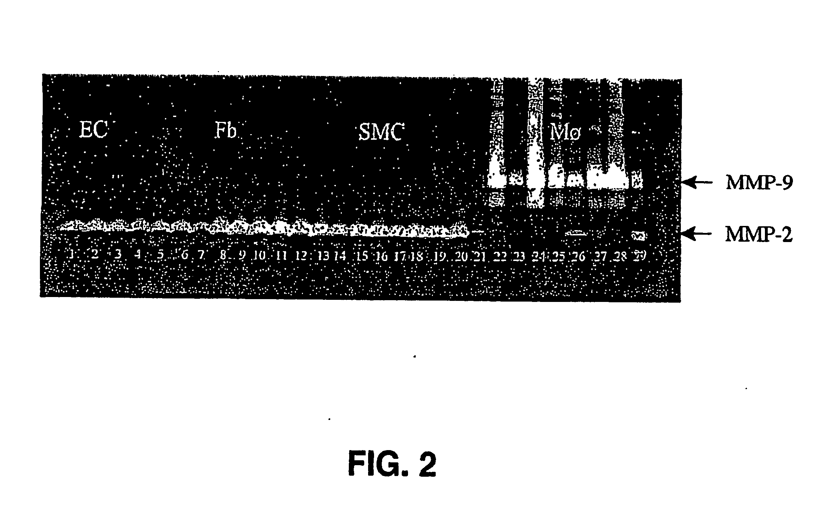

FIG. 2 illustrates a zymogram developed to determine the levels of MMP-2 and MMP-9 secreted by EC, SMC, Fb, and MΦ cultured on various protein coatings. Gelatin zymography was run on conditioned medium from EC, SMC, Fb, and MΦ cultured on collagen I (lanes 1,8,15,22), collagen IV (lanes 2,9,16,23), laminin (lanes 3,10,17,24), fibronectin (lanes 4,11,18,25), fibrin (lanes 5,12,19,26), plastic (lanes 6,13,20,27) and BSA-blocked plastic (lanes 7,14,21,28). Pro-MMP-2 was secreted by EC (lanes 1-7), Fb (lanes 8-14), and SMC (lanes 15-21) at constant levels. The band of MMP-2 activity in the MΦ samples (lanes 22-28) seen in lane 26 is not derived from the cells, but rather is intrinsically present in fibrin preparations as shown on cell free control. Two forms of MMP-9 - Pro (92 kDa) and active (82 kDa) - appear only in the MΦ samples (lanes 22-28). These results demonstrate that MMP-2 is secreted exclusively by EC, SMC, and Fb, and that none of these cells secrete MMP-9. Conversely, MΦ secretes MMP-9, but no MMP-2. The only lane where MMP-2 appeared in MΦ derived media (lane 26) was shown to be the result of contamination by the protein coating fibrin with this MMP, and can be discounted.

As shown in FIG. 2, the protease present in different cell types is distinct and can be determined experimentally using techniques known by those skilled in the art. More specifically, FIG. 2 shows that MMP-9 is present in macrophages, but not the other three cell types evaluated, all of which contain MMP-2. Additional zymography, conducted on macrophage samples from 18 human donors, confirmed this finding, as only one of the 18 donors showed significant production of MMP-2 in 2 out of 3 MΦ cultures, and this patient had a substance abuse problem with diazepam that may have resulted in unusual physiochemistry. Utilizing this information in the preparation of a biomaterial according to the present invention, a polymer matrix 10 can be prepared that would be degraded by cells containing MMP-2 (EC, SMC, and Fb), but would prevent entry by MMP-9 containing cells (MΦ), if a protease substrate 14 was incorporated into the matrix that was selectively cleaved by MMP-2.

Additional methodologies can be utilized by those skilled in the art to determine the proteases present in different cell types. One methodology that has the advantage of providing a more global picture of the proteases present within a cell type is gene arrays. Gene array experiments are sometimes referred to as "reverse Northerns". In Northern blots, RNA is blotted onto a filter and hybridized with a probe to detect a particular species of mRNA as a distinct band or spot. In gene array hybridization, cDNAs are spotted onto a filter or slide and hybridized with a probe made from an mRNA population. Usually, probes are made by reverse-transcribing mRNA into single-stranded cDNA in the presence of labeled nucleotides. The labeled probe, therefore, is a population of cDNA molecules representing the original mRNA population. Probes are hybridized with filters containing cDNAs spotted in a 2-dimensional array. The amount of hybridization to a given clone represents the amount of mRNA present for the corresponding gene.

Use of gene arrays, also called microarrays, can provide the mRNA expression levels of up to 10,000 genes simultaneously, though microarrays allowing for the simultaneous analysis of up to 100 genes are more typical. An example of a microarray that is commercially available is the GEArray™ Q Series Extracellular Matrix & Adhesion Molecules Gene Array, manufactured by SuperArray Bioscience Corporation, that contains 96 genes encoding for cell adhesion and extracellular matrix proteins. Among the proteins detected by this assay are numerous matrix metalloproteinases, serine proteinases, and cysteine proteinases. This particular array allows for the determination of the mRNA expression levels of 80% of currently known MMPs and 12 other proteases, generating nearly a complete protease profile for cell types of interest. This information enables preparation of biomaterials that are susceptible to penetration by numerous specific cell types, based on the particular protease or proteases present in the cells of interest.

Proteases, like most enzymes, are highly specific both in terms of the reaction catalyzed and in their choice of reactants, which are referred to as substrates. The degree of specificity for substrates is usually high and sometimes virtually absolute. This is due to the fact that much of the catalytic power of enzymes comes from their bringing substrates together in a favorable orientation in enzyme-substrate complexes, which can only occur if the substrate has a structure that corresponds to structure of the enzyme active site. There arc specific peptide sequences which will fit the active site and be catalytically cleaved by proteases present in the cell types. Thus, once the protease profile of the cell type has been determined, the next step in preparing a cell-specific biomaterial is to identify the substrate or substrates that are cleaved by the proteases present. Research by Smith et al. has determined that hexapeptides generally provided superior MMP substrates, although larger and smaller peptides will often provide a cleavage site. See Smith et al., "Rapid Identification of Highly Active and Selective Substrates for Stromelysin and Matrilysin using Bacteriophage Peptide Display Libraries" J. Biol. Chem. 270, 6440 (1995).

A powerful tool for the evaluation of numerous peptide sequences uses phage display libraries. Phage display libraries use phage material (viruses) that infect a recombinant DNA host such as *E*. *Coli.* Because of its accessibility to solvent, a peptide displayed on a phage particle often behaves essentially as it would if free in solution, allowing it to be studied by a variety of chemical techniques. For a review of phage display systems, see Smith, G. and Petrenko, V., "Phage Display", Chem. Rev. 97, 391 (1997). Phage display libraries are useful for determining the amino acid sequence for protease substrates cleaved by the proteases that have been found to be present in preferred cells.

Recent studies using phage display libraries have surprisingly shown that a high degree of selectivity can be achieved for distinct MMPs using specific hexapeptide substrates. Chen *et al*. investigated the substrates specific for MMP-2 in Chen et al. "A Unique Substrate Recognition Profile for Matrix Metalloproteinase-2", J. Biol. Chem. 277, 6, 4485 (2002). Activated MMP-2 was used to select optimal substrates from a phage display library. Following phage hydrolysis and subsequent amplification, several individual phage clones were selected randomly and assessed for hydrolysis by MMP-2. Cleavage within the peptide hexamer substrate was gauged by measuring the liberation of a FLAG epitope that is positioned to the N-terminal side of the substrate. Thirty individual clones were selected for sequencing based on their cleavage, and four distinct groups of substrates were found, as shown in Table I.

**Table I. Sequences of phage substrates for MMP-2**

| | Peptide Sequence | | | | | | | | | % Hydrolysis | | |
|---|---|---|---|---|---|---|---|---|---|---|---|---|
| Group | SEQ ID NO: | | | P₃ | P₂ | P₁ | P_{1'} | P_{2'} | P_{3'} | Clone | exp 1 | exp 2 |
| **I** | 1 | S | G | **P** | Y | V | **I** | W | L | A45 | 100 | 100 |
| | 2 | R | P | **P** | M | A | **K** | T | A | A50 | 100 | 100 |
| | 3 | S | G | **P** | V | W | **Y** | M | S | A26 | 100 | 98 |
| | 4 | S | G | **P** | V | R | **Y** | M | V | A35 | 100 | 90 |
| | 5 | G | L | **P** | R | W | **L** | L | T | A47 | 91 | 90 |
| | 6 | G | R | **P** | I | R | **M** | K | T | A46 | 51 | 49 |
| | | | | | | | | | | | | |
| **II** | 7 | L | R | **L** | A | A | **I** | T | A | B49 | 100 | 100 |
| | 8 | I | Y | **L** | G | W | **A** | T | A | A29 | 100 | 100 |
| | 9 | P | M | **I** | S | V | **L** | T | A | A54 | 98 | 96 |
| | 10 | E | S | **L** | A | Y | **Y** | T | A | B74 | 81 | 90 |
| | 11 | G | S | **L** | H | S | **I** | I | T | B54 | 72 | 75 |
| | 12 | S | D | **I** | R | M | **L** | T | A | B79 | 64 | 70 |
| | 13 | F | N | **L** | Y | N | **L** | T | A | B66 | 51 | 50 |
| | 14 | G | Y | **L** | Q | V | **L** | L | T | B46 | 48 | 50 |
| | 15 | S | G | **I** | V | N | **L** | Y | P | A43 | 38 | 40 |
| | 16 | E | Y | **L** | H | M | **R** | T | A | A10 | 36 | 40 |
| | 17 | V | G | **L** | I | A | **I** | T | A | A6 | 30 | 44 |
| | | | | | | | | | | | | |
| **III** | 18 | R | S | **L** | S | R | **L** | T | A | C9 | 100 | 100 |
| | 19 | N | R | **Y** | S | S | **L** | T | A | A34 | 100 | 100 |
| | 20 | W | T | **S** | S | W | **L** | T | A | A31 | 100 | 100 |
| | 21 | G | A | **V** | S | W | **L** | L | T | A13 | 100 | 93 |
| | 22 | A | N | **I** | S | D | **L** | T | A | B37 | 97 | 95 |
| | 23 | T | I | **L** | S | L | **L** | T | A | B53 | 73 | 75 |
| | 24 | G | F | **N** | S | M | **L** | K | T | C3 | 28 | 30 |
| | | | | | | | | | | | | |
| **IV** | 25 | G | L | **H** | R | R | **I** | D | T | C5 | 60 | n.d. |
| | 26 | G | M | **H** | S | R | **P** | P | T | A9 | 50 | 63 |
| | 27 | H | M | **H** | K | A | **L** | T | A | A21 | 30 | 49 |
| | 28 | G | R | **H** | L | G | **L** | Q | T | A4 | 30 | 45 |
| | 29 | G | L | **H** | K | K | **V** | H | T | C6 | 19 | n.d. |
| | 30 | G | A | **H** | A | K | **H** | W | T | D3 | 13 | n.d. |

The first group of substrates contains the P-X-X↓X_{Hy} motif, where X_{Hy} represents a large hydrophobic residue. This motif is a substrate for a number of different MMPs, as mentioned above. Substrates in groups II-IV represent recognition motifs for MMP-2. Groups II substrates contain the I/L X-X-X_{Hy} motif, in which the last hydrophobic residue is usually the amino acid Ile or Leu. Group III substrates contain the X_{Hy}-S-X-L motif, where Ser and Leu are invariant. Group IV substrates are comprised of the H-X-X-X_{Hy} motif, which is similar to the cleavage site for MMP-2 within laminin-5.

Representative peptides from groups I-IV were synthesized by Chen et al. and used to characterize substrate hydrolysis in greater detail, as shown in Table II. The cleavage position within these peptides was determined by mass spectrometry. The peptides were also used to determine the selectivity of the substrates for individual MMPs. The rate of hydrolysis of each peptide by MMP-2, MMP-9, MMP-7, and MMP-13 was determined and compared. The initial velocity of hydrolysis was measured across a range of peptide concentrations and reciprocal plots were used to derive *K*ₘ and *k*_{cat} for MMP-2, and the *k*_{cat}/*K*ₘ ratio for each enzyme. When cleaved by MMP-2, most of the substrates exhibited Michaelis constants in the mM range, with turnover rates ranging from 1 to 750 s⁻¹. Correspondingly, the *k*_{cat}/*K*ₘ ratios were all between 1.6 x 10⁴ M⁻¹s⁻¹ and 2 x 10⁵ M⁻¹s⁻¹. Substrates from groups II, III, and IV exhibited *k*_{cat}/*K*ₘ ratios that are 8 to 350-fold higher for MMP-2 than for the other MMPs. As a group, the substrates within group II show the most selectivity.

Tables I and II were both prepared by Chen et al, as found in Chen et al. "A Unique Substrate Recognition Profile for Matrix Metalloproteinase-2", J. Biol. Chem. 277, 6, 4485 (2002), but have been modified to provide appropriate sequence identification information.

**Table II. Measuring peptides hydrolysis by a panel of MMPs.**

| | | | **MMP-2** | | | **MMP-9** | **MMP-7** | **MMP-13** |
|---|---|---|---|---|---|---|---|---|
| **Peptide** | **SEQ ID NO:** | **Sequence** | ***k_{cat}*** **(s⁻¹)** | ***Kₘ*** **(mM)** | **k_{cat}/Kₘ (M⁻¹s⁻¹)** | ***k_{cat}*/*Kₘ* (M⁻¹s⁻¹)** | ***k_{cat}*/*Kₘ* (M⁻¹s⁻¹)** | ***k_{cat}*/*Kₘ* (M⁻¹s⁻¹)** |
| **Group** I | | | | | | | | |
| A3 | 31 | SGAKPRA ↓ LTA | 400 | 3.6 | 1.1E+05 | 4.9E+04 | 5.2E+03 | 7.9E+03 |
| **Group II** | | | | | | | | |
| B49 | 32 | SGLRLAA ↓ ITA | 510 | 4.5 | 1.1E+05 | 7.9E+03 | 1.9E+04 | 8.5E+03 |
| B74 | 33 | SGESLAY ↓ YTA | 622 | 2.2 | 2.8E+05 | 2.6E+03 | 7.9E+02 | 2.0E+03 |
| B74P | 34 | SGESPAY ↓ YTA | 264 | 0.6 | 4.4E+05 | 3.0E+04 | 1.5E+03 | 5.3E+03 |
| B74R | 35 | SGESLRY ↓ YTA | 61 | 0.8 | 7.6E+04 | 1.0E+05 | 7.1E+02 | 1.1E+03 |
| C9 | 36 | SGRSLSR ↓ LTA | 740 | 4.4 | 1.7E+05 | 8.6E+02 | 4.8E+03 | 3.3E+03 |
| C9R | 37 | SGRSLRR ↓ LTA | 302 | 6.4 | 4.7E+04 | 5.3E+04 | 2.2E+03 | 3.9E+03 |
| **Group III** | | | | | | | | |
| A13 | 38 | SGAVSW ↓ LLTA | 105 | 1.3 | 8.1E+04 | 5.4E+03 | 6.4E+03 | 2.9E+03 |
| A13P | 39 | SGAPSW ↓ LLTA | 204 | 0.4 | 5.1E+05 | 1.7E+04 | 5.4E+03 | 4.9E+03 |
| A13R | 40 | SGAVRW ↓ LLTA | 28 | 0.4 | 7.0E+04 | 2.0E+05 | 3.5E+03 | 2.9E+03 |
| B37 | 41 | SGANISD ↓ LTA | 353 | 2.4 | 1.5E+05 | 2.1E+03 | 8.6E+03 | 2.6E+03 |
| A34 | 42 | SGNRYSS ↓ LTA | 202 | 2.2 | 9.2E+04 | 2.3E+03 | 1.1E+03 | 3.9E+03 |
| **Group IV** | | | | | | | | |
| A21 | 43 | SGHMHKA↓ LTA | 124 | 7.7 | 1.6E+04 | 2.1E+03 | n.d. | n.d. |
| A21A | 44 | SGHMHAA↓ LTA | 601 | 3.2 | 1.9E+05 | 1.9E+03 | n.d | n.d. |

| | | | | | | | | |
|---|---|---|---|---|---|---|---|---|
| n.d. = not determined 5 Kridel *et al*. investigated the substrates specifically cleaved by MMP-9 in Kridel et al. "Substrate Hydrolysis by Matrix Metalloproteinase-9", J.-Biol. Chem. 276, 23, 20572 (2001). Random hexamer | | | | | | | | |

substrates were again displayed on the surface of a phage using the phage display library method. The library included 2.4 x 10⁸ independent transformants, giving a 75% confidence that each of the 6.4 x 10⁷ possible random hexamer sequences were represented in the library. Optimal substrates were then selected by exposing the phage library to a recombinant form of the catalytic domain of MMP-9 expressed in HEK 293 cells. Phage selection was performed with three rounds of exposure to active MMP-9, after which individual phage clones were selected for sequencing. Three basic groups of substrate motifs were discovered, as shown in Table III.

**Table III. Sequence alignment and grouping of phage selected by MMP-9**

| Group | SEQ ID NO: | Peptide Sequence | | | | | | | | | Clone | % Hydrolysis | |
|---|---|---|---|---|---|---|---|---|---|---|---|---|---|
| | | | | | | | | | | | | 1 | 2 |
| **I** | 45 | | | P | L | F | Y | S | V | | A10 | 100 | 100 |
| | 46 | K | I | P | R | T | L | T | | | A11 | 100 | 100 |
| | 47 | | | P | L | R | L | S | W | | A12 | 100 | 100 |
| | 48 | | | P | R | A | V | S | T | | A6 | 100 | 99 |
| | 49 | K | G | P | R | Q | I | T | | | C15 | 100 | 98 |
| | 50 | | | P | R | P | L | S | G | | D12 | 82 | 94 |
| | 51 | | W | P | L | G | L | A | | | D35 | 89 | 91 |
| | 52 | F | R | P | R | S | I | T | | | D36 | 87 | 91 |
| | 53 | R | L | P | V | G | L | T | | | D20 | 89 | 90 |
| | 54 | R | S | P | K | S | L | T | | | D21 | 88 | 90 |
| | 55 | | | P | V | W | L | A | A | | D22 | 85 | 90 |
| | 56 | I | H | P | S | S | L | T | A | | D4 | 87 | 89 |
| | 57 | | | P | A | S | F | T | S | | D29 | 87 | 86 |
| | 58 | G | Q | P | H | Y | L | T | | | B1 | 79.6 | 85 |
| | 59 | M | K | P | A | S | W | T | | | B2 | 77.5 | 77 |
| | 60 | T | H | P | Y | T | M | T | | | B10 | 74.1 | 82 |
| | 61 | | T | P | A | Y | M | L | T | | A13 | 78.9 | 72 |
| | 62 | | | P | L | Y | L | T | | | D2 | 79 | 82 |
| | 63 | | | P | G | L | I | G | T | | D1 | 75 | 85 |
| | 64 | | | P | R | S | I | S | N | | D24 | 82 | 82 |
| | 65 | R | L | P | A | S | Y | T | | | D30 | 82 | 60 |
| | 66 | N | P | P | R | Y | L | T | | | D5 | 70 | 67 |
| | 67 | | | P | K | T | Q | I | S | | D8 | 73 | 65 |
| | 68 | S | V | P | R | H | F | T | | | D17 | 70 | 52 |
| | 69 | L | L | P | A | W | L | T | | | D3 | 56 | 48 |
| | 70 | T | H | P | Y | T | M | T | | | D14 | 50 | 47 |
| | 71 | L | R | P | A | K | S | T | | | A18 | 28.9 | 30 |
| | 72 | S | G | P | S | T | S | T | | | A9 | 13.1 | 5.8 |
| | | | | | | | | | | | | | |
| **Consensus** | | | | P | R | S/T | Hy | S/T | | | | | |
| **II** | 73 | | | G | S | G | L | K | A | | A7 | 79.8 | 76 |
| | 74 | | | A | M | G | L | K | S | | B9 | 73.3 | 86 |
| | 75 | | | K | V | G | L | R | T | | B8 | 13.3 | 12 |
| | | | | | | | | | | | | | |
| **IIA** | 76 | | | G | R | R | L | I | H | H | C11 | 94.9 | 74 |
| | 77 | | H | P | R | R | S | I | T | | C1 | 13.9 | 29 |
| | 78 | | | G | R | R | L | L | S | R | A16 | 20.2 | 11 |
| | 79 | | A | L | R | R | L | E | T | | C13 | 8.3 | 24 |
| | 80 | | F | Y | K | R | V | L | T | | B6 | 11.7 | 11.1 |
| | 81 | | | F | R | R | I | C | V | | C7 | 17.9 | 0 |
| | 82 | V | F | F | R | R | Q | T | A | | C8 | 8.9 | 0 |
| | | | | | | | | | | | | | |
| **IIIB** | 83 | G | L | A | R | N | I | T | A | | B3 | 66 | 70 |
| | 84 | F | G | S | R | Y | L | T | A | | A19 | 45 | 50 |
| | 85 | | Q | D | R | Y | L | N | T | | C6 | 40 | 27 |

A kinetic analysis was run using 10 peptides, representing each of the three groups of substrates, as shown in Table IV. Kinetic analysis identified the position of the scissile bonds and demonstrated the selective cleavage of these peptide substrates by comparing the rates of cleavage by MMP-7 and MMP-13. Most of the substrates tested were cleaved more efficiently by MMP-9. The *k_{cat}*/*Kₘ* ratios ranged from 2.6- to 47-fold higher for MMP-9 than for either MMP-7 or MMP-13. Tables III and IV were obtained from Kridel et al. "Substate Hydrolysis by Matrix Metalloproteinaso-9", J. BioL Chem. 276, 23, 5 20572 (2001), with slight modification.

**Table IV. Kinetic analysis of MMP-9 peptide substrates based on phage clones**

| Peptide | SEQ ID NO: | Sequence | MMP-9 | | | MMP-13 | MMP-7 |
|---|---|---|---|---|---|---|---|
| | | | *k_{cat}*/*Kₘ* | *k_{cat}* | | *k_{cat}*/*Kₘ* | *k_{cat}*/*Kₘ* |
| | | | *M⁻¹S⁻¹* | *S⁻¹* | *mM* | *M⁻¹S⁻¹* | *M⁻¹S⁻¹* |
| C15 | 86 | *SGKGPRQ* ↓ *ITA* | 188,000 | 703 | 3.7 | 15,400 | 13,800 |
| A11 | 87 | *SGKIPRT* ↓ *LTA.* | 67,500 | 86.4 | 1.3 | 23,500 | 7,000 |
| A6 | 88 | *SGPRA* ↓ *VSTTA* | 61,000 | 178 | 2.9 | 12,400 | 1,300 |
| A11m1 | 89 | *SGKIPRR* ↓ *LTA* | 160,000 | 224 | 1.4 | 15,200 | 24,000 |
| A11m2 | 90 | *SGKIPRT* ↓ *ATA* | 2000 | 9.4 | 4.7 | ** | ** |
| A10 | 91 | *SGPLF* ↓ *YSVTA* | 25,600 | 20 | 0.5 | ** | ** |
| B1 | 92 | *SGQPHY* ↓ *LTTA* | 8800 | 9 | 1 | ** | ** |
| A18 | 93 | *SGLRPAK* ↓ *STA* | 5800 | 78 | 13.5 | ** | ** |
| A7 | 94 | *SG* ↓ *LKALMITA* | 12,400 | 30 | 2.4 | 12,300 | 41,000 |
| A19 | 95 | *SGFGSRY* ↓ *LTA* | 13,200 | 11 | 0.8 | * | 1,000 |
| A16 | 96 | *SGRR* ↓ *LLSRTA* | 1200 | ** | ** | ** | ** |
| C11 | 97 | *SGRR* ↓ *LIHHTA* | * | ** | ** | ** | ** |

Studies similar to those described above have also been conducted for MMP-13 and MMP-14. See, respectively, Deng. et al., "Substrate Specificity of Human Collagenase 3 assessed using a Phage-displayed Peptide Library" J. Biol. Chem. 275, 40, 31422 (2000), and Kridel et al., "A Unique Substrate Biding Mode Discriminates Membrane Type-1 Matrix Metalloproteinase from Other Matrix Metalloproteinases" J. Biol. Chem. 277,26,23788 (2002).

These studies, together with those described above, provide extensive information on substrates useful as protease substrates 14 that enable cell-specific degradation of the polymer matrix 10. In particular, the specificity of MMPs for specific peptide sequences has been clearly demonstrated. Those skilled in the art will appreciate that the methods disclosed above can be utilized to create biomaterial tailored to permit cell-specific entry for any cell type carrying proteases distinct from those present in other cell types.

### Biomaterial with Adhesive Proteins

The biomaterial of the present invention also preferably utilizes adhesion sites 16 attached to the hydrophilic polymer 12. Adhesion sites 16 are short peptide sequences that are attached to the polymer and serve to encourage the migration and adhesion of the cell type 18. These adhesion sites 16 are present on the surface of the polymeric matrix 10 and are also generally present in the interior, where they serve to secure and encourage the ingrowth of the cell type 18 subsequent to degradation of the protease substrates 14 and concurrent degradation of the polymer matrix 10.

A variety of adhesion-promoting peptides have been identified as being the active domains of adhesion promoting proteins such as fibronectin, vitronectin, laminin, collagen, von Willebrand factor, and osteonectin. These peptides, referred to herein as adhesion sites 16, can be readily attached to the hydrophilic polymer 12, by means that will be discussed below. A partial list of the peptides that can serve as adhesion sites 16 is provided in Table V. This list was assembled by Yamada et al. in Yamada Y., and Klienman H.K., "Functional domains of cell adhesion molecules", Curr. Opin. Cell. Biol. 4,819 (1992). These peptides are useful for affecting a variety of cellular reactions, such as cell migration, attachment, and the induction of particular cell phenotypes, such as stimulating macrophages to release beneficial growth factors rather than forming foreign body giant cells.

**Table V. Cell Binding Domain Sequences of Extracellular Matrix Proteins**

| Protein | Sequence* | SEQ ID NO: | Role |
|---|---|---|---|
| Fibronectin | RGDS | 98 | Adhesion of most cells, via α₅β₁ |
| | LDV | | Adhesion |
| | REDV | 99 | Adhesion |
| Vitronectin | RGDV | 100 | Adhesion of most cells, via αᵥβ₃ |
| Laminin A | LRGDN | 101 | Adhesion |
| | IKVAV | 102 | Neurite extension |
| Laminin B1 | YIGSR | 103 | Adhesion of many cells, via 67 kD laminin receptor |
| | PDSGR | 104 | Adhesion |
| Laminin B2 | RNIAEIIKDA | 105 | Neurite extension |
| Collagen I | RGDT | 106 | Adhesion of most cells |
| | DGEA | 107 | Adhesion of platelets, other cells |
| Thrombospondin | RGD | | Adhesion of most cells |
| | VTXG | 108 | Adhesion of platelets |

We desire to utilize adhesion-promoting peptides that not only promote cell adhesion, but selectively promote adhesion and migration of preferred cells. Three peptides in particular; RGD, PHSRN (SEQ. ID NO: 109), and YIGSR (SEQ. ID NO: 103), selectively affect the adhesion and migration of microvascular endothelial cells (MVEC) and human vascular smooth muscle cells (HVSMC) and are useful in this invention. The tripeptide RGD, present in all major extracellular matrix proteins, has been shown to enhance the adhesion and spread of Fb, EC, and SMC when the peptide is grafted onto surfaces. PHSRN, another cell binding sequence found in fibronectin, stimulates invasion of serum-free extracellular matrices by keratinocytes and fibroblasts, and enhances the re-epithelialization and contraction of dermal wounds. YIGSR (SEQ. ID NO: 103), a peptide derived from the laminin B 1 chain, is part of a class of adhesive peptides that, unlike RGD and PHSRN (SEQ. ID NO: 109), does not interact with the integrin family of cell receptors, but instead with laminin binding protein (LBP). This peptide promotes adhesion and spreading of a large number of cell types, including EC, Fb, and SMC.

Multiple adhesion peptides may be used to increase cell adhesion and selectivity. This was demonstrated using polyethylene glycol (PEG) hydrogels modified with RGD and PHSRN (SEQ. ID NO: 109) or YIGSR (SEQ. ID NO: 103). The effect of adhesion and spreading of MVEC and HVSMC on hydrogels derivatized with one or more of these peptides is illustrated in FIG.s 3a and 3b. On PEG derivatized with RGD, both cell types achieved maximal adherence at 0.32 pmol/cm² of RGD loading on PEG hydrogel, and a gradual decline thereafter. For PHSRN (SEQ. ID NO: 109) and YIGSR-(SEQ. ID NO: 103) bearing PEG, both cell types showed cell adhesion that gradually increased with increasing peptide concentration, although the overall binding for both peptide-derived hydrogels was significantly reduced compared to hydrogel derivatized with RGD. When hydrogels were derivatized with equimolar amounts of PHSRN (SEQ. ID NO: 109) or YIGSR (SEQ. ID NO: 103) in combination with RGD (at a level of 1.24 pmol/cm² of each peptide), different effects were observed for the addition of PHSRN (SEQ. ID NO: 109) versus YIGSR (SEQ. ID NO: 103), and for HVSMC and MVEC adhesion. HVSMC showed a significant increase in adhesion on hydrogel coated with RGD and YIGSR, while showing a decrease in adhesion to hydrogel coated with RGD and PHSRN (SEQ. ID NO: 109), both relative to hydrogel coated with RGD alone. The adhesion of MVEC, on the other hand, was not significantly altered by either combination of peptides relative to RGD alone. While not intending to be bound by theory, the lack of synergism in adhesion observed for use of PHSRN (SEQ. ID NO: 109) with RGD may be due to the requirement for precise spacing between the two peptides necessary to obtain this effect.

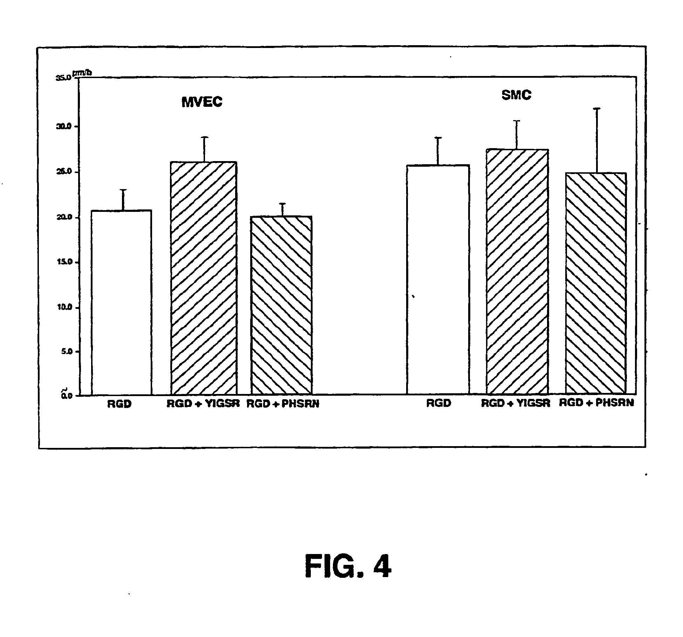

Multiple adhesion peptides may be utilized to increase cell migration in a selective fashion. The effect of coating hydrogel with PHSRN (SEQ. ID NO: 109) or YIGSR (SEQ. ID NO: 103) in combination with RGD on MVEC and HVSMC is shown in FIG. 4. Time lapse video microscopy was used to determine the migration speed of individual cells moving on PEG hydrogels derivatized with various peptides. A migration speed of 20.3 µm/h was observed for MVEC on RGD-derivatized PEG. The addition of an equimolar amount of YIGSR (SEQ. ID NO: 103) resulted in an increased speed of migration, to 25.3 µm/h, whereas the addition of an equimolar amount of PHSRN (SEQ. ID NO: 109) did not affect MVEC mobility. Addition of equimolar amounts of either YIGSR (SEQ. ID NO: 103) or PHSRN (SEQ. ID NO: 109) to RGD-derivatized PEG did not significantly affect the mobility of HVSMC. Comparing the migration rates of the two cell types on RGD plus YIGSR (SEQ. ID NO: 103) or PHSRN (SEQ. ID NO: 109) to that on RGD alone, only MVEC showed a significant difference in motility. While not intending to be bound by theory, it is noteworthy that the YIGSR peptide (SEQ. ID NO: 103) has been found to co-localize LBP with a-actinin and vinculin, two crucial components of focal adhesion sites. As RGD modified surfaces encourage formation of focal adhesion sites with concomitant cell spreading, YIGSR (SEQ. ID NO: 103) may influence the rate of assembly and disassembly of focal adhesion sites by driving the association of LBP with RGD containing focal adhesion sites.

### Synthesis of the Peptide-Containing Polymer Matrix

The polymer matrix 10 may include several components that are connected together to form a biomaterial that encourages cell-specific ingrowth. The polymer matrix 10 comprises a hydrophilic polymer 12 including one or more attached peptide sequences. The peptide sequences are either protease substrates 14 or adhesion sites 16, or both protease substrates 14 and adhesion sites 16. As can be seen in FIG. 1, the protease substrates 14 are used to cross-link the polymer, while the adhesion sites 16 are found at the ends of hydrophilic polymer 12 branches or chains. The protease substrate 14 should cross-link portions of polymer matrix since this leads to degradation of the polymer matrix 10 upon cleavage by cellular proteases. Adhesion sites 16 may be placed in a number of locations, but are generally not used to cross-link portions of the polymer matrix 10. The adhesion sites 16 may be placed at the end of polymer chains, as shown, or they may be derivatized to the polymer chain, or they may also be positioned as cross-linking peptides. It is preferable that the adhesion sites 16 be scattered throughout the polymer matrix 10 so that ingrowth and cell migration are encouraged as new surfaces are exposed by polymer degradation; however, simple derivatization to the surface of the matrix will still result in cell-specific adhesion and migration.

The peptide sequences may be linked to the polymer matrix by various techniques known to those skilled in the art. The majority of these techniques involve the use of linking groups added to the ends of the peptide and/or polymer strands that provide complementary reactive sites, such as those used in conventional peptide synthesis. Care is taken when reacting the peptides with the polymer matrix that reaction takes place at the ends of the peptides and desirably not with any free amines that happen to be present within the peptide, such as those provided by asparagine or glutamine. If necessary, protective group chemistry can be employed to shield these side chain amines prior to reaction of the end groups.

A preferred method of linking the peptide sequences to the polymer matrix is by nucleophilic addition reactions.: A first compound - either the peptide or polymer - is provided, having α-β-unsaturated moieties. The second compound - a peptide or a polymer, depending on what was used as the first compound - is provided, having nucleophilic moieties. Together, the first and second compounds provide both the peptide and polymer components. The reaction may be conducted such that the nucleophile is placed on the polymer and the unsaturated group on the peptide sequences, or vice versa. The nucleophilic addition reaction results in a polymer matrix 10 including peptide sites that are either adhesion sites 16 or protease substrates 14, or both. For example, while a PEG-triacrylate will react appropriately with a dicysteine-containing peptide sequence, likewise a PEG-trithiol will react with a diacrylate-containing peptide sequence. Combining the nucleophilic peptide sequences with the polymer derivatized with unsaturated groups results in an addition reaction that combines the two structures. If cross-linking is desired, nucleophiles can be supplied at both ends of the peptide sequences. Providing a branched polymer with more than two reactive sites will result in a branched structure.

A particularly preferred means of joining peptides to the polymer matrix is the use of Michael-type addition reactions, which are the 1,4 addition of a nucleophile to an α-β-unsaturated carbonyl compound. Michael-type addition to unsaturated groups can take place in good yields at room temperature and under mild conditions using a wide variety of nucleophiles, as is known in the art. Generally, the unsaturated structure is used to derivatize the terminal ends of the polymer to form a site where a nucleophile can bind. Desirably, reaction conditions should be such that amine groups present in the peptides do not react with α-β-unsaturated groups, as this would alter the peptide structure and reduce its ability to act as an adhesion or protease cleavage site.

The unsaturated group-introducing compound can be, for example, acryloyl chloride, methacryloyl chloride, acrylic acid, methacrylic acid, or isocynatate having an unsaturated, e.g., α,β-unsaturated group at one end of the molecule, e.g., isocyanatoethyl methacrylate. Particular nucleophiles and unsaturated groups useful for this type of reaction are disclosed in M. Lutolf, et al., "Systematic modulation of Michael-type reactivity of thiols through the use of charged amino acids" Bioconjug. Chem. 12, 1051-1056 (2001).

The hydrophilic polymer 12 used in the present invention preferably forms a hydrogel. While the present invention is not restricted to the use of hydrogel, the hydrophilic polymer 12 is desirably a biocompatible polymer that can be derivatized with peptide sequences that encourage cell-specific ingrowth once so derivatized by encouraging the selective adhesion and migration of preferred cells into the polymer matrix 10. Polyethylene glycol (PEG) provides a monomer useful for the preparation of a hydrogel polymer. One can readily purchase or synthesize linear or branched PEG hydrogel. The polymer may also be other synthetic polymers, such as poly(ethylene oxide), poly(vinyl alcohol), poly(vinyl pyrrolidone), poly(acrylic acid), poly(ethoxazoline) or poly(ethylene oxide)-co-poly(propyline oxide) block copolymers, or mixtures thereof. Other polymers, copolymers, block copolymers, or graft polymers suitable for use as the hydrophilic polymer 12 can be used, as would be clear to one skilled in the art.

In addition to hydrophilic polymer 12, one or more peptide sequences are needed to function as protease substrates 14 and/or adhesive sites 16. Exemplary peptide sequences providing the desired activity as protease substrates 14 or adhesives sites 16 are described above, and shown in the tables. For attachment to the hydrophilic polymer 12, the peptide sequence includes reactive sites. As noted above, the reactive site may be a nucleophile or an unsaturated group susceptible to nucleophilic addition. Nucleophiles that engage in Michael addition reactions and their appropriate reactive counterparts are preferred. This results in peptides with a structure such as H₂N-NuXXXXXX-COOH for peptides that are intended to attach at one point only, and structures such as H₂N-NuXXXXXXNu-COOH, where Nu represents a compound bearing a suitable nucleophile, and X represents other amino acids. A preferred amino acid bearing a nucleophile is cysteine, in which the nuclophile is a sulfhydryl group. The number of amino acids (X) shown above represents a hexapeptide; however, a variety of lengths of peptides are suitable for use. A peptide is defined, for present purposes, as two or more amino acids residues linked by a peptide bond, (i.e., an amide linkage). While the present invention can utilize a long peptide sequence such as that forming an entire protein, the phrase "peptide sequence", as used herein, generally refers to a fragment of a protein rather than an entire protein. Peptide sequences may be purchased commercially, isolated through biochemical methods known to those skilled in the art, or prepared using peptide synthesis techniques, including the use of automated peptide synthesizers.

An overall process for preparation of the polymer matrix 10 may include the following steps. A polymer of a chosen molecular weight is derivatized with a group that will react by nucleophilic addition. This may involve reacting polyethylene glycol with acryloyl chloride, for example, to provide double bond unsaturation points at the ends of the PEG chains. The derivatized polymer may then be reacted with preferably less than one equivalent of adhesive peptide that contains a structure that will react with the compounds used to derivatize the polymer. The adhesive peptide may be a peptide strand with an amino acid sequence known to cause adhesion by cell types, referred to herein as adhesion sites 16. For example, this can be the peptide GCGGGRGDSPG (SEQ. ID NO: 110), which contains a single thiol-bearing cysteine and the adhesive sequence RGD. The thiol provides the nucleophile in this particular example. Preferably, less than one equivalent of the adhesive peptide is provided, as this will leave unreacted derivatives for subsequent attachment by the cross-linking protease substrate peptide. The derivatized polymer with attached adhesive peptide may be reacted with the protease substrate peptide. The protease substrate peptide bears an amino acid sequence susceptible to proteolytic cleavage by a limited set of one or more proteases, and further contains at least two reactive sites for use in attaching the protease substrate to the polymer. For example, this can be the peptide GCRDSGESLAYYTADRCG (SEQ ID NO: 113), that contains two thiol-bearing cysteines that bear sulfhydryl group nucleophiles, and an MMP-2 protease substrate sequence.

### Applications for Biomaterial providing Cell-specific Ingrowth

The peptide-including polymer matrix can be used for a variety of different applications. Given the flexibility of the matrix to be tailored to encourage cell ingrowth by a variety of cell types, the various applications to which this biomaterial could be used are nearly endless. As such, the applications listed are exemplary, and should not be considered limiting.

One application for the cell-specific ingrowth matrix of the present invention is use as a coating for an implantable medical device. As noted above, even inert material will eventually provoke an undesired response from surrounding healthy tissue, such as inflammation or encapsulation, since the cells in healthy tissue are in active communication through the extracellular matrix. However, if a medical device is coated with a biomaterial of the present invention, the biomaterial may allow ingrowth of desired cells, creating a region around the device where the distinction between the implant and body tissue is blurred. This blunts the response of the body to the implanted device, and promotes a healing response instead of the adverse response to a foreign body.

Surfaces to be coated may include, for example, metals and alloys, ceramics, glasses, and polymers. Examples of metals include stainless steel, titanium, nickel, cobalt, chrome, niobium, molybdenum, zirconium, tantalum, and combinations thereof. Ceramic materials, such as alumina and zirconia, glasses, and calcium phosphates, such as hydroxyapatite and tricalcium phosphate, may also be coated in accordance with the invention. The biomaterial can further be applied to various polymers and plastics, more preferably biocompatible or bioresorbable polymers such as polylactic acid or polyglycolic acid, but also polyolefins, such as polyethylene and the like.

The surface to be coated may be a flat, dense or have a complex shape. It may have a porous, beaded or meshed ingrowth surface, depending on its intended function. In particular, the surface may be that of a medical device which is to be used in vivo. Examples of medical devices and medical materials that may be coated according to the invention include, but are not limited to, catheters, fibers, non-woven fabrics, vascular grafts, porous metals such as those used to reconstruct the acetabulum in revision, dental filling materials, materials for approximation, adhesion of tissues, materials used in osteo-synthesis (e. g. pins or bone screws), cardiac patches, sutures, soft and hard tissue scaffolds and fillers (e. g. collagen, calcium phosphate, bioglass), stents, bone void fillers intended for the repair of bone defects, intrauterine devices, root canal fillers, drug delivery pumps, implantable infusion pumps, spacer devices, implants containing medicinal products, and scaffolds for tissue engineering.

A coating of biomaterial may be applied to a surface by methods known to those skilled in the art, such as brushing, spraying, wiping, dipping, extruding or injecting. The latter three methods are preferred when porous structures or fibrous meshes are to be coated. Use of these methods allows penetration by the biomaterial into the pores of the devices and a provides a substantially uniform coating of the total surface area. Brushing and spraying are preferred in case of non-porous devices.

A related application of the cell-specific ingrowth matrix of the invention is for use on the surface of an implantable vascular device such as a vascular graft, heart valve, or sewing ring. Conventional implantable vascular devices do not generally stimulate sufficient ingrowth to allow for proper healing and a satisfactory long-term outcome. A vascular graft bearing a matrix encouraging cell-specific ingrowth, on the other hand, facilitates integration by encouraging infiltration of the graft by cell types such as endothelial cells and smooth muscle cells, while discouraging the ingrowth of generally non-desired cell types such as macrophages, which are part of the immune response to foreign material. Ingrowth and a healing response may be particularly advantageous in the case of biodegradable vascular devices where ingrowth can eventually lead to complete replacement of the medical device with natural tissue.

The polymer matrix may also be utilized as a tissue scaffolding material. Tissue scaffolding is a type of tissue engineering, in which naturally occurring and/or synthetic materials are used in conjunction with cells to create biologic substitutes that serve as functional tissue replacements. In tissue scaffolding, the material is used to provide a scaffold on which natural, healthy tissue can develop. It can be used for the creation of heart tissue, blood vessels, cartilage, bone and many other structures of the human body, including whole organs for transplantation. Tissue scaffolding utilizes the cell-specific ingrowth matrix of the present invention in a fashion similar to that described for vascular grafts. Namely, the biomaterial encourages the growth of desired cell types, leading to a healing response and/or replacement of the biomaterial with natural tissue. As tissue scaffolding is utilized to recreate a variety of tissue and organs, recruitment of desired cell types can be particularly advantageous in providing the cells necessary for the particular tissue that is being engineered. Nerve regeneration, angiogenesis, and skin, bone, and cartilage repair are all examples of tissue engineering that can benefit from cell-specific ingrowth utilizing the present invention.

The polymer matrix of the present invention may also be utilized in a drug delivery context. Biologically active agents such as drugs that can be incorporated into the coating may vary widely in nature; in principle any type of agent may be incorporated as long as its nature or used amount does not obstruct the desired function of the biomaterial. As the biomaterial is biodegradable in vivo by specific cells, and allows diffusion of molecules, the biologically active agent will be released to the surroundings of the coating in a controlled manner. These agents may be added to the solution in amounts ranging from 0 to 50 wt. %, preferably from 1 to 20 wt.%.

The biologically active agent or agents may be selected from a wide variety of active agents, such as a natural products, synthetic drugs, growth factors, or genetic materials. The agent may be released by diffusive mechanisms or cell-specific degradation of the biomaterial described above. Release of the agent through degradation of the biomaterial is particularly important, as this mechanism takes advantage of the cell-specific nature of the biomaterial and allows for synergistic effects in which drug is released to further stimulate the healing response.

The term "biologically active agent", as used herein, means an agent which provides a therapeutic or prophylactic effect. Such agents include, but are not limited to, antimicrobial agents (including antibacterial and antifungal agents), anti-viral agents, antitumor agents, hormones, immunogenic agents, growth factors, lipids, and lipopolysaccharides. Biologically active agents which may be incorporated further include, but are not limited to, non-peptide, non-protein low molecular weight drugs. These generally have a molecular weight which is less than 1500, and in particular less than 500 daltons. A second important group of biologically active agents are biologically active peptides and proteins.

In addition to the specific applications described above, the biomaterial of the present invention is contemplated as being used for two or more applications simultaneously. For example, the biomaterial of the present invention could be used to coat the surface of a vascular graft and release beneficial drug upon degradation of the matrix by desired cell-types.

The compositions, devices, and methods described herein will be more fully understood with reference to the following non-limiting examples.

### Example 1

### Preparation of starting materials: acrylated polyethylene glycol and peptide sequences

Polyethylene glycol (PEG, 20 kDa, 8-arm, Shearwater Corporation) was acrylated by treatment with acryloyl chloride in accordance with Elbert et al., Protein Delivery from Materials Formed by Self-selective Conjugate Addition Reaction, J Control Release 2001; 76:11-25. A 10% PEG solution in toluene was dried by azeotropic distillation (removing a third of the volume) and diluted back to original concentration through anhydrous addition of dichloromethane (DCM). After cooling in an ice bath and addition of 50% molar excess of triethylamine (TEA), 50% molar excess of acryloyl chloride (AcCl) was added dropwise over 5 minutes. The ice-bath was removed, and the reaction continued under an Argon atmosphere at room temperature (RT) for 24 hours. Subsequent filtering, precipitation (3 times into cold hexane), extraction from an aqueous solution (10% PEG-8Ac, 0.5% NaCl, pH = 6) into DCM, final precipitation (hexane) and drying (in vacuo, 24 hours, RT) resulted in the desired product. The complete acrylation of the polymer was confirmed by NMR spectroscopy (Varian 400 MHz), based on the absence of a -CH₂-OH peak in the ¹³C spectrum and on the 103% acrylation efficiency calculated from the ¹H peaks at 3.3-4.3 ppm (97.76 H, -CH₂-CH₂-O-CO-CH=CH₂) and 5.8 ppm (dd, 0.45H, -CO-CH=CH_{cis}Hₜᵣₐₙₛ).

Peptides were synthesized by solid-state chemistry on resin using an automated peptide synthesizer (Perceptive Biosystems, Farmington, MA), with standard 9-fluorenylmethyloxycarbonyl (Fmoc) chemistry. As an example, the following adhesive peptides were synthesized. GCGGGRGDSPG (SEQ ID NO:110) (based on the RGD peptide), GCGGGVPHSRNSG (SEQ ID NO: 111) (based on the PHSRN peptide), and GCGGGYIGSRG (SEQ ID NO: 112) (based on the YIGSR peptide). Peptides were purified by C-18 chromatography (Perceptive Biosystems Biocad 700E) and analyzed by MALDI-TOF mass spectrometry.

### Example 2

### Preparation of PEG hydrogel containing adhesive peptides

Hydrogel derivatized with adhesive peptides was prepared for use in testing the adhesion of MVEC and HVSMC. 20 kDa PEG-8-Ac hydrogels (12.5% w/v, before swelling) were bound to either a single peptide at 0, 0.5, 2, 8, and 32 mol percent (using RGD, PHSRN (SEQ ID NO: 109), or YIGSR (SEQ ID NO: 103) peptides) or a combination of RGD (8 mol% of the available acrylate groups) with an equimolar loading of either PHSRN (SEQ ID NO: 109) or YIGSR (SEQ ID NO: 103). Linear 3.4 kDa PEG-dithiol was subsequently used to cross-link the peptide-capped PEG-8Ac.

PEG-8Ac acrylate (50% of final volume), peptides (according to the dilution series described above, 25% of final volume) and PEG-2SH (25% of final volume) were dissolved in 50 mM Phosphate Buffered Saline (PBS), vortexed, and sterilized by filtration (0.45 µm). PEG-8Ac and peptide aliquots were mixed and incubated for 1h at 37°C. The reaction between PEG-acrylates and cysteine-containing peptides has been shown to go to completion. After coupling the peptide to the PEG-8Ac, the PEG-2SH was added and the complete mixture immediately aliquoted into a 96 well dish (40 µl/well). After incubation for 1 h at 37°C, the cross-linked hydrogels were allowed to swell for 24 hours in 50 mM PBS and then rinsed 3 times with PBS. The final peptide concentration, expressed in pmol/cm², was calculated to be equivalent to the number of peptide molecules in the top 10 nm of the hydrogel after swelling. The 0, 0.5, 2, 8 and 32 mol percent peptide loadings thus correspond to 0, 0.08, 0.32, 1.24 and 3.9 pmol/cm²

### Example 3

### Preparation of PEG hydrogel cross-linked by protease substrate peptides

The peptide GCRDSGESLAYYTADRCG (SEQ ID NO: 113) has been shown to be sensitive to hydrolysis by MMP-2, and contains more than one thiol (present in cysteine) for use as a nucleophile in addition reactions with unsaturated groups. The peptide was synthesized according to the methods described above. Hydrogel was formed from PEG-2500-3A, in which the molecular weight notations refers to total average molecular weight. A polymer matrix was then formed from PEG-2500-3A and GCRDSGESLAYYTADRCG (SEQ ID NO: 113). Hydrogel polymer matrices were formed in 10 mM phosphate buffered saline with triethanolamine to adjust the pH to 8.0-9.0 as tested by paper pH strips (hydrogel formation reactions were performed at 50 microliter and smaller scales). Hydrogels have been made by either predissolving the peptide and then adding peptide solution to PEG-3A, presissolving the PEG-3A and adding its solution to the peptide, or by predissolving both solutions and then mixing them in appropriate ratios. Reference is made to Elbert et al., PCT/US00/02608, published August 3, 2000 as Publication No. WO 00/44808.

A 5.0 mg quantity of GCRDSGESLAYYTADRCG (SEQ ID NO: 113)and a 7.0 mg quantity of PEG-2500-3A were weighed separately. 62 microliters of PBS•TEA (10 mM PBS with 13 microliters of triethanolamine/ml) were added to the PEG-2500-3A to give a solution of 4.4 mg/40 microliters. The buffer volume added to the PEG-2500-3A was always adjusted to give the same final concentration on a mass/volume basis. The PEG solution was allowed to sit until the PEG-3A had dissolved, and then 40 microliters of the PEG-3A solution were added to the peptide. The mixture was then briefly stirred. The desired peptide-containing hydrogel formed after 20-30 minutes.

### Example 4

### Preparation of PEG hydrogel including adhesive peptides and cross-linked protease substrate peptides

Hydrogel containing both adhesive peptides and cross-linked protease substrates are prepared. 20 kDa PEG-8-Ac hydrogels (12.5% w/v, before swelling) are bound to an adhesive peptide to 0, 0.5, 2, 8, and 32 mol percent (using RGD, PHSRN, or YIGSR peptides). The peptide GCRDSGESLAYYTADRCG (SEQ ID NO: 113) (that contains two thiol-bearing cysteines) are subsequently used to crosslink the peptide-capped PEG-8Ac.

PEG-8Ac acrylate (50% of final volume), adhesive peptides (according to the dilution series described above, 25% of final volume) and GCRDSGESLAYYTADRCG (SEQ ID NO: 113) (25% of final volume) are dissolved in 50 mM Phosphate Buffered Saline (PBS), vortexed, and sterilized by filtration (0.45 µm). PEG-8Ac and peptide aliquots are mixed and incubated for 1h at 37°C. The reaction between PEG-acrylates and cysteine containing peptides is known to go to completion. After coupling the peptide to the PEG-8Ac, the GCRDSGESLAYYTADRCG (SEQ ID NO: 113) is added and the complete mixture immediately aliquoted into a 96 well dish (40 µl/well). After incubation for 1 h at 37°C, the cross-linked hydrogels are allowed to swell for 24 hours in 50 mM PBS and then rinsed 3 times with PBS.

### Example 5

### Adhesion and Migration of MVEC and HVSMC on hydrogel matrix containing adhesive peptides

Hydrogel matrices were prepared including RGD, PHSRN (SEQ ID NO: 109), or YIGSR (SEQ ID NO: 103), and combinations with RGD, as described in Example 2. Cells were harvested enzymatically (using 0.05% Trypsin/0.02% EDTA in PBS, pH 7.4), resuspended in serum-free medium (MCDB 131, 0.1 % BSA, 1% ITS, 5 ng/ml bFGF), seeded onto hydrogel matrix (20,000 cells in 150 µl medium), and allowed to adhere for 5 hours. Cell adhesion was quantified photometrically by determination of AlamarBlue™ (Biosource Inc.) reduction. The hydrogel matrices were rinsed 3 times before AlamarBlue™ containing medium (1% v/v, medium as above) was added (150 µl/well). After 6 hours, 90 ml/well of the supernatant was transferred to new 96 well plates and read at 560 nm and 590 nm to determine the amount of reduced AlamarBlue™ present. Results were calculated according to a standard curve of cells on tissue culture treated plastic. Results are shown above in FIG.s 3a and 3b.

Migration of MVEC and HVSMC was studied on PEG-8Ac after coupling RGD alone 98 mol%) or in combination with equimolar amounts of either PHSRN (SEQ ID NO: 109) or YIGSR (SEQ ID NO: 103). Hydrogels (50 µl) were produced in an identical manner to that employed in the adhesion study, with the exception that they were cast between 2 glass plates (0.4 mm spacing).

The resulting hydrogel discs were transferred into a 12-well tissue culture plate and immobilized at the bottom of the plate using Teflon rings. Cells were harvested enzymatically (0.05% Trypsin/0.02% EDTA in PBS, pH 7.4), resuspended in serum-free medium (MCDB 131, 0.1% BSA, 1% ITS, 5 ng/ml bFGF), seeded onto the hydrogel discs (2,200 cells/cm² in 1.5 ml medium), and allowed to adhere for 6h before the plate was placed within a computer controlled climatised chamber (PeCon GmbH, Erbach-Back, Germany) maintained at 5% CO₂, 37°C and 100% humidity) onto the microscope stage. Single cell migration was quantified using a time-lapse videomicroscopy setup on an inverted light microscope with computerized stage (Leica DM IRBE microscope, x and y stage; Leica microscopes, Wetzlar, Germany). A videocamera (Sony XC-75E; Sony Corporation, Tokyo, Japan) on the microscope was interfaced with a frame grabber board (Meteor II, Matrox Graphics Inc., Dorval, Canada) of the controlling PC, allowing for software-controlled picture acquisition. Two macros were written for the Leuica QWIN image software. The first allowed quantification of cell locomotion from the resulting stack of 41 pictures at each location by calculating the movement of the cell nucleus for every 15 min. interval. Finally, migration speed was determined from the sum of quarter-hourly distances of cell nuclei divided by the total time. The results are shown in FIG. 4.

### Example 6

### Preferred cell ingrowth into peptide-containing hydrogel

Hydrogel matrices can be evaluated for their ability to encourage cell-specific ingrowth by utilized the three-dimensional spheroid model of endothelial cell differentiation developed by Korff and Augustin, as described in Korff, T. and Augustin, H., "Tensional forces in fibrillar extracellular matrices control directional capillary sprouting" J. Cell Sci. 112, 3249 (1999).

Two hydrogel matrices could be prepared as described in Example 4. One hydrogel matrix (HM-EC) prepared would be designed to encourage endothelial cell ingrowth, in which the adhesive peptide is GCGGGRGDSPG (SEQ ID NO: 110) (based on the RGD peptide), and the protease substrate peptide is GCRDSGESLAYYTADRCG (SEQ ID NO: 113). These are peptides that are adhesive for endothelial cells, and are cleaved by proteases (MMP-2, specifically) present in those endothelial cells. Another hydrogel matrix (HM-MΦ) would be prepared to encourage macrophage cell ingrowth. In this hydrogel matrix, the adhesive peptide is GCGGGRGDSPG (SEQ ID NO: 110), and the protease substrate peptide is GCKGPRQITCG (SEQ ID NO: 114). These peptides are adhesive for macrophages, and cleaved by proteases (MMP-9, specifically) present in macrophage cells.

Spheroids of endothelial cells and macrophage cells are then prepared. Endothelial cell spheroids with 750 cells are suspended in culture medium containing 25% carboxymethylcellulose and seeded in nonadherent round-bottom 96 well plates. Under these conditions, suspended cells contribute to the formation of a single EC spheroid. The standardized spheroids should be harvested within 24 hours. A similar procedure is used to prepare macrophage spheroids.

Growth of macrophage spheroids would then be compared with endothelial spheroids in both HM-EC and HM-MΦ. In order to do this, Macrophage or Endothelial cell spheroids are embedded into both hydrogel matrices, and incubated at 37°C in 5% CO₂ at 100% humidity. The cumulative length of all capillary-like sprouts originating from the central plain of an individual sphereoid is measured at 100x magnification using a digitized imaging system (DP-Soft, Olympus) connected to an inverted microscope (IX50, Olympus). At least 10 spheroids per experimental group and experiment should be analyzed. This analysis takes into consideration that the ingrowth induced by the nature of the hydrogel matrix is reflected by the length of the individual capillary-like sprouts as well as the number of capillary like sprouts. For morphological analysis, the hydrogel matrixes are fixed for 24 hours in HBSS containing 4% formaldehyde, after which they are processed for paraffin embedding. Following dehydration (in a graded series of ethanol and isopropanol, 24 hours each; 4°C), the gels are immersed with paraffin I (melting temperature 42°C) for 24 hours at 60°C and paraffin II (melting temperature 56°C) for 36 hours at 70°C. The resulting paraffin block is cooled to room temperature and trimmed for sectioning. Sections should be stained with hematoxylin.

Results are expected to indicated that while there is significant ingrowth by EC spheroids in HM-EC, there is little ingrowth by EC in HM-MΦ. Likewise, there should be significant ingrowth by macrophage spheroids in HM-MΦ, but little ingrowth by macrophages into HM-EC. These results would demonstrate the ability of the hydrogel matrix to encourage cell specific ingrowth by two different cell types.

## Claims

1. A polymer matrix capable of being selectively degraded by a desired, predetermined cell type, the polymer matrix comprising: a hydrophilic polymer crosslinked by a peptide sequence including a protease cleavage site, and wherein the protease cleavage site is a substrate for a matrix metalloproteinase present in said predetermined cell type, but absent or present in much lower levels in non-desired cells, and wherein the protease cleavage site is selectively cleaved by the matrix metalloproteinase.

2. The polymer matrix of claim 1, wherein the non-desired cells are macrophages and/or neutrophils.

3. The polymer matrix of claim 1 or 2, wherein the predetermined cell type is endothelial cells, smooth muscle cells, or fibroblasts.

4. The polymer matrix of any one of claims 1 to 3, further comprising an appended peptide sequence containing an adhesion site that increases the affinity of the polymer matrix for the predetermined cell type.

5. The polymer matrix of any one of claims 1 to 4, wherein the hydrophilic polymer is a hydrogel polymer.

6. The polymer matrix of any one of claims 1 to 5, wherein the hydrophilic polymer is selected from the group consisting of poly(ethylene glycol), poly(ethylene oxide), poly(vinyl alcohol), poly(vinyl pyrrolidone), poly(acrylic acid), poly(ethyloxazoline) and poly(ethylene oxide)-co-poly(propylene oxide) and mixtures thereof.

7. The polymer matrix of any one of claims 1 to 6, wherein the peptide sequence including a protease cleavage site comprises a matrix metalloproteinase-2 substrate.

8. The polymer matrix of any one of claims 4 to 7, wherein the adhesion site is selected from the group of the peptide RGD and peptides having SEQ ID NO: 109 and SEQ ID NO: 103.

9. The polymer matrix of any one of claims I to 8, further comprising a biologically active agent.

10. A biocompatible medical device, comprising: an implantable medical device having a surface at least partially coated with a polymer matrix as claimed in any one of claims 1 to 9.

11. The biocompatible medical device of claim 10, wherein the implantable medical device comprises a vascular graft, a heart valve, or a sewing ring.

12. The biocompatible medical device of claim 10 or 11, wherein the protease cleavage site is a substrate for a matrix metalloproteinase present in a cell type that promotes tissue healing by ingrowth into the polymer matrix.

13. The biocompatible medical device of claim 12, wherein the tissue healing comprises transmural vascularization of the implanted medical device.

14. An implantable film comprising a polymer matrix as claimed in any one of claims 1 to 9.

15. The implantable film of claim 14, wherein the film is capable of preventing the formation of surgical adhesions.

16. The implantable film of claim 14 or 15, wherein the film comprises a tissue scaffold.

17. The implantable film of any one of claims 14 to 16, wherein the protease cleavage site is a substrate for a matrix metalloproteinase present in a cell type that promotes tissue healing by ingrowth into the polymer matrix.

18. A method for preparing an implantable medical device capable of integrating into a mammalian body by encouraging ingrowth by a predetermined cell type, comprising:
a) preparing a polymer matrix as claimed in any one of claims 1 to 9; and
b) coating the polymer matrix on at least a portion of the medical device.

19. The method of claim 18, wherein the hydrophilic polymer bonds to said peptide sequences containing a protease cleavage site by nucleophilic addition between a strong nucleophile and an alkene.

20. The method of claim 19, wherein the strong nucleophile is a thiol or an amine.

21. The method of claim 19, wherein the alkene is an acrylate or a quinone.

22. In combination, a polymer matrix as claimed in any one of claims 1 to 9, and cells of said predetermined cell type in contact with said polymer matrix.

23. A method of providing cell-specific ingrowth of cells into a polymer matrix, comprising contacting with cells of a predetermined cell type with a polymer matrix as claimed in any one of claims 1 to 9, with the proviso that said method is not a method for treatment of the human or animal body by surgery or therapy or a diagnostic method practised on the human or animal body.

24. The method of claim 23 further including the step of providing said polymer matrix onto the surface of an implantable medical device.

## Patentansprüche

1. Eine Polymermatrix, die in der Lage ist, durch eine gewünschte, vorbestimmte Zellart selektiv abgebaut zu werden, wobei die Polymermatrix folgendes umfasst: ein hydrophiles Polymer, das durch eine Peptidsequenz vernetzt ist, die eine Protease-Spaltstelle einschließt, und wobei die Protease-Spaltstelle ein Substrat für eine Matrixmetalloproteinase ist, die in der vorbestimmten Zellart vorliegt, jedoch in nicht-gewünschten Zellen fehlt oder bei viel geringeren Konzentrationen vorliegt, und wobei die Protease-Spaltstelle durch die Matrixmetalloproteinase selektiv gespalten wird.

2. Die Polymermatrix nach Anspruch 1, wobei die nicht-gewünschten Zellen Makrophagen und/oder Neutrophile sind.

3. Die Polymermatrix nach Anspruch 1 oder 2, wobei die vorbestimmte Zellart Endothelzellen, Glattmuskelzellen oder Fibroblasten ist.

4. Die Polymermatrix nach einem der Ansprüche 1 bis 3, die ferner eine angehängte Peptidsequenz umfasst, die eine Adhäsionsstelle enthält, die die Affinität der Polymermatrix für die vorbestimmte Zellart erhöht.

5. Die Polymermatrix nach einem der Ansprüche 1 bis 4, wobei das hydrophile Polymer ein Hydrogel-Polymer ist.

6. Die Polymermatrix nach einem der Ansprüche 1 bis 5, wobei das hydrophile Polymer aus der Gruppe ausgewählt ist, die aus Poly(ethylenglycol), Poly(ethylenoxid), Poly(vinylalkohol), Poly(vinylpyrrolidon), Poly(acrylsäure), Poly(ethyloxazolin) und Poly(ethylenoxid)-co-poly(propylenoxid) und Gemischen davon besteht.

7. Die Polymermatrix nach einem der Ansprüche 1 bis 6, wobei die Peptidsequenz, die eine Protease-Spaltstelle einschließt, ein Matrixmetalloproteinase-2-Substrat umfasst.

8. Die Polymermatrix nach einem der Ansprüche 4 bis 7, wobei die Adhäsionsstelle aus der Gruppe des Peptids RGD und Peptiden, die die SEQ-ID NR: 109 und SEQ-ID NR: 103 aufweisen, ausgewählt ist.

9. Die Polymermatrix nach einem der Ansprüche 1 bis 8, die ferner einen biologisch wirksamen Stoff umfasst.

10. Eine biokompatible medizinische Vorrichtung, die folgendes umfasst: eine implantierbare medizinische Vorrichtung mit einer Oberfläche, die mindestens teilweise mit einer Polymermatrix, wie nach einem der Ansprüche 1 bis 9 beansprucht, beschichtet ist.

11. Die biokompatible medizinische Vorrichtung nach Anspruch 10, wobei die implantierbare medizinische Vorrichtung ein Gefäßtransplantat, eine Herzklappe oder einen Nähring umfasst.

12. Die biokompatible medizinische Vorrichtung nach Anspruch 10 oder 11, wobei die Protease-Spaltstelle ein Substrat für eine Matrixmetalloproteinase ist, die in einer Zellart vorliegt, die eine Gewebeausheilung durch Einwachsen in die Polymermatrix fördert.

13. Die biokompatible medizinische Vorrichtung nach Anspruch 12, wobei die Gewebeausheilung eine transmurale Vaskularisation der implantierten medizinischen Vorrichtung umfasst.

14. Eine implantierbare Folie, die eine Polymermatrix, wie nach einem der Ansprüche 1 bis 9 beansprucht, umfasst.

15. Die implantierbare Folie nach Anspruch 14, wobei die Folie in der Lage ist, die Bildung von chirurgischen Adhäsionen zu verhindern.

16. Die implantierbare Folie nach Anspruch 14 oder 15, wobei die Folie ein Gewebekonstrukt umfasst.

17. Die implantierbare Folie nach einem der Ansprüche 14 bis 16, wobei die Protease-Spaltstelle ein Substrat für eine Matrixmetalloproteinase ist, die in einer Zellart vorliegt, die eine Gewebeausheilung durch Einwachsen in die Polymermatrix fördert.

18. Ein Verfahren zum Herstellen einer implantierbaren medizinischen Vorrichtung, die zur Integration in einen Säugetierkörper in der Lage ist, indem das Einwachsen durch eine vorbestimmte Zellart angeregt wird, wobei das Verfahren folgendes umfasst:
a) Herstellen einer Polymermatrix, wie nach einem der Ansprüche 1 bis 9 beansprucht; und
b) Auftragen der Polymermatrix auf mindestens einen Teil der medizinischen Vorrichtung.

19. Das Verfahren nach Anspruch 18, wobei das hydrophile Polymer an die Peptidsequenzen, die eine Protease-Spaltstelle enthalten, durch nucleophile Addition zwischen einem starken Nucleophil und einem Alken bindet.

20. Das Verfahren nach Anspruch 19, wobei das starke Nucleophil ein Thiol oder ein Amin ist.

21. Das Verfahren nach Anspruch 19, wobei das Alken ein Acrylat oder ein Chinon ist.

22. In Kombination, eine Polymermatrix, wie nach einem der Ansprüche 1 bis 9 beansprucht, und Zellen der vorbestimmten Zellart in Kontakt mit der Polymermatrix.

23. Ein Verfahren zum Bereitstellen eines zellspezifischen Einwachsens von Zellen in eine Polymermatrix, das folgendes umfasst: Kontaktieren mit Zellen einer vorbestimmten Zellart mit einer Polymermatrix, wie nach einem der Ansprüche 1 bis 9 beansprucht, mit der Maßgabe, dass das Verfahren kein Verfahren zum Behandeln eines menschlichen oder tierischen Körpers durch Chirurgie oder Therapie oder ein diagnostisches Verfahren ist, das an dem menschlichen oder tierischen Körper durchgeführt wird.

24. Das Verfahren nach Anspruch 23, das ferner den Schritt des Bereitstellens der Polymermatrix auf der Oberfläche einer implantierbaren medizinischen Vorrichtung einschließt.

## Revendications

1. Matrice polymère apte à être dégradée de façon sélective par un type de cellule prédéterminé, désiré, la matrice polymère comprenant : un polymère hydrophile réticulé par une séquence peptidique comprenant un site de clivage de protéase, et le site de clivage de protéase étant un substrat pour une métalloprotéinase de matrice présente dans ledit type de cellule prédéterminé, mais absente ou présente dans des taux très inférieurs dans des cellules non désirées, et le site de clivage de protéase étant clivé de façon sélective par la métalloprotéinase de matrice.

2. Matrice polymère selon la revendication 1, dans laquelle les cellules non désirées sont des macrophages et/ou des neutrophiles.

3. Matrice polymère selon l'une des revendications 1 ou 2, dans laquelle le type de cellule prédéterminé consiste en cellules endothéliales, cellules de muscle lisse ou fibroblastes.

4. Matrice polymère selon l'une quelconque des revendications 1 à 3, comprenant en outre une séquence peptidique attachée contenant un site d'adhésion qui augmente l'affinité de la matrice polymère pour le type de cellule prédéterminé.

5. Matrice polymère selon l'une quelconques des revendications 1 à 4, dans laquelle le polymère hydrophile est un polymère hydrogel.

6. Matrice polymère selon l'une quelconques des revendications 1 à 5, dans laquelle le polymère hydrophile est choisi dans le groupe constitué par le poly(éthylène glycol), le poly(oxyde d'éthylène), le poly(alcool vinylique), la poly(vinyl pyrrolidone), le poly(acide acrylique), la poly(éthyloxazoline) et le poly(oxyde d'éthylène)-co-poly(oxyde de propylène) et leurs mélanges.

7. Matrice polymère selon l'une quelconque des revendications 1 à 6, dans laquelle la séquence peptidique comprenant un site de clivage de protéase comprend un substrat de métalloprotéinase-2 de matrice.

8. Matrice polymère selon l'une quelconque des revendications 4 à 7, dans laquelle le site d'adhésion est choisi dans le groupe du peptide RGD et des peptides ayant SEQ ID NO : 109 et SEQ ID NO : 103.

9. Matrice polymère selon l'une quelconques des revendications 1 à 8, comprenant en outre un agent biologiquement actif.

10. Dispositif médical biocompatible comprenant : un dispositif médical implantable ayant une surface au moins partiellement revêtue pour une matrice polymère telle que définie à l'une quelconque des revendications 1 à 9.

11. Dispositif médical biocompatible selon la revendication 10, dans lequel le dispositif médical implantable comprend une greffe vasculaire, une valvule cardiaque ou un anneau de couture.

12. Dispositif médical biocompatible selon l'une des revendications 10 ou 11, dans lequel le site de clivage de protéase est un substrat pour une métalloprotéinase de matrice présente dans un type de cellule qui favorise la cicatrisation des tissus par croissance interne dans la matrice polymère.

13. Dispositif médical biocompatible selon la revendication 12, dans lequel la cicatrisation des tissus comprend une vascularisation transmurale du dispositif médical implanté.

14. Film implantable comprenant une matrice polymère telle que définie à l'une quelconque des revendications 1 à 9.

15. Film implantable selon la revendication 14, dans lequel le film est capable d'empêcher la formation d'adhérences chirurgicales.

16. Film implantable selon l'une des revendications 14 ou 15, dans lequel le film comprend un échafaudage tissulaire.

17. Film implantable selon l'une quelconque des revendications 14 à 16, dans lequel le site de clivage de protéase est un substrat pour une métalloprotéinase de matrice présente dans un type de cellule qui favorise la cicatrisation des tissus par croissance interne dans la matrice polymère.

18. Procédé de préparation d'un dispositif médical implantable apte à s'intégrer dans un corps de mammifère par encouragement de la croissance interne par un type de cellule prédéterminé, comprenant :
a) la préparation d'une matrice polymère telle que définie à l'une quelconque des revendications 1 à 9 ; et
b) l'application en revêtement de la matrice polymère sur au moins une partie du dispositif médical.

19. Procédé selon la revendication 18, dans lequel le polymère hydrophile se lie auxdites séquences peptidiques contenant un site de clivage de protéase par addition nucléophile entre un nucléophile puissant et un alcène.

20. Procédé selon la revendication 19, dans lequel le nucléophile puissant est un thiol ou une amine.

21. Procédé selon la revendication 19, dans lequel l'alcène est un acrylate ou une quinone.

22. En combinaison, matrice polymère selon l'une quelconque des revendications 1 à 9, et cellules dudit type de cellule prédéterminé en contact avec ladite matrice polymère.

23. Procédé pour assurer une croissance interne spécifique d'une cellule de cellules dans une matrice polymère, comprenant la mise en contact de cellules d'un type de cellule prédéterminé avec une matrice polymère telle que définie à l'une quelconque des revendications 1 à 9, à la condition que ledit procédé ne soit pas un procédé pour le traitement du corps humain ou animal par chirurgie ou thérapie ou un procédé de diagnostic pratiqué sur le corps humain ou animal.

24. Procédé selon la revendication 23 comprenant en outre l'étape de disposition de ladite matrice polymère sur la surface d'un dispositif médical implantable.
